(19) **Europäisches Patentamt / European Patent Office / Office européen des brevets**

(11) **EP 4 575 914 A1**

(12) # EUROPEAN PATENT APPLICATION
published in accordance with Art. 153(4) EPC

(43) Date of publication:
**25.06.2025 Bulletin 2025/26**

(21) Application number: **22955465.4**

(22) Date of filing: **20.09.2022**

(51) International Patent Classification (IPC):
***G06N 3/12*** (2023.01)

(52) Cooperative Patent Classification (CPC):
**G06F 3/06; G06N 3/12; G06N 3/123**

(86) International application number:
**PCT/CN2022/119796**

(87) International publication number:
**WO 2024/036689 (22.02.2024 Gazette 2024/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2022 CN 202210988065**

(71) Applicant: **Digicodon Technologies Co., Ltd.**
**Hangzhou, Zhejiang 310059 (CN)**

(72) Inventor: **ZHANG, Lushuai**
**Hangzhou, Zhejiang 310059 (CN)**

(74) Representative: **Torggler & Hofmann**
**Patentanwälte - Innsbruck**
**Torggler & Hofmann Patentanwälte GmbH & Co KG**
**Wilhelm-Greil-Straße 16**
**6020 Innsbruck (AT)**

(54) **METHOD, DEVICE AND SYSTEM FOR STORING INFORMATION IN MOLECULES**

(57)     The present disclosure provides a method, a device, and a system for storing information in a molecule. The method includes: obtaining information to be stored, wherein the information to be stored has one or more bit-groups, a position of each bit-group in the information to be stored is represented by a first address, a value of each bit-group is represented by a first content, and each bit-group has one or more bits; determining a molecule module corresponding to at least one bit-group of the one or more bit-groups, wherein the molecule module includes a first molecule module, and the first molecular module is configured to represent both the first address and the first content of a corresponding bit-group; and generating a composition based on the determined molecular module such that the composition corresponds to the information to be stored.

Fig.1

## Description

## CROSS-REFERENCE TO RELATED APPLICATION

This application claims priority to Chinese Patent Application No. 202210988065.6, entitled "METHOD, DEVICE AND SYSTEM FOR STORING INFORMATION IN MOLECULE", filed on August 17, 2022, the disclosure content of which is incorporated herein by reference in its entirety.

## TECHNICAL FIELD

The present disclosure relates to the field of storage technology, in particular to a method, a device and a system for storing information in a molecule.

## BACKGROUND

With the substantial development of information technology, people's demand for data storage is increasing rapidly. Traditional data storage media include hard disks, flash memories, magnetic tapes, optical disks, etc., which have problems such as low storage density, short storage time, and high energy consumption. In order to achieve a higher storage density and a more reliable storage effect, it is now possible to store information in a molecule.

## SUMMARY

In related technology, there is still no suitable solution for storing information in a molecule that can be integrated with a computer system in terms of coding efficiency, writing speed and cost. Therefore, there is a need for an improvement to the existing molecular storage technology.

Thus, the embodiments of the present disclosure propose the following solutions.

According to an aspect of the embodiments of the present disclosure, a method for storing information in a molecule is provided, including:

 obtaining information to be stored, wherein the information to be stored has one or more bit-groups, a position of each bit-group in the information to be stored is represented by a first address, a value of each bit-group is represented by a first content, and each bit-group has one or more bits;
 determining a molecular module corresponding to at least one bit-group of the one or more bit-groups, wherein the molecular module includes a first molecular module, and the first molecular module is configured to represent both the first address and the first content of a corresponding bit-group; and
 generating a composition based on the determined molecular module such that the composition corresponds to the information to be stored.

In some embodiments, obtaining the information to be stored includes:

 dividing initial information to be stored to generate one or more pieces of information to be stored, wherein a number of bits of each piece of information to be stored is less than a number of bits of the initial information to be stored, and the number of bits of each piece of information to be stored is equal or not equal to each other; or
 combining a plurality of pieces of initial information to be stored to generate the information to be stored, wherein the number of bits of the information to be stored is greater than the number of bits of each piece of initial information to be stored.

In some embodiments, determining the molecular module corresponding to the at least one bit-group of the one or more bit-groups includes:
determining the first molecular module corresponding to each bit-group of the one or more bit-groups.

In some embodiments, the information to be stored has a plurality of bit-groups, and values of the plurality of bit-groups contain at least two kinds of first contents, and
determining the molecular module corresponding to the at least one bit-group of the one or more bit-groups includes:
determining that a bit-group with a value of one kind of first content does not correspond to any molecule module, and determining the first molecule module corresponding to at least one bit-group of other bit-group with a value of other kind of first content.

In some embodiments, determining the molecular module corresponding to the at least one bit-group of the one or more bit-groups includes:

 determining if a second bit-group and a first bit-group of the one or more bit-groups satisfy a preset relationship, wherein the first address of the second bit-group is different from the first address of the first bit-group; and
 in a case that the second bit-group and the first bit-group satisfy the preset relationship, determining a molecular module corresponding to the second bit-group, wherein the molecular module includes a second molecular module, and the second molecular module is configured to represent the preset relationship between the corresponding second bit-group and the first bit-group.

In some embodiments, the second molecular module includes at least one of:

 a stroke incremental module, wherein the stroke incremental module corresponding to a first preset number $a_1$ is configured to represent that the second bit-group and the first bit-group satisfy the following preset relationship: there are $a_1$ consecutive second bit-groups immediately after the first bit-group, and the first content of each second bit-group is same as the first content of the first bit-group, $a_1$ being a positive integer;

a stroke multiple module, wherein the stroke multiple module corresponding to a second preset number $a_2$ is configured to represent that the second bit-group and the first bit-group satisfy the following preset relationship: there are $(a_2-1)$ consecutive second bit-groups immediately after the first bit-group, and the first content of each second bit-group is same as the first content of the first bit-group, $a_2$ being a positive integer greater than 1;

a stroke flip module, wherein the stroke flip module corresponding to a third preset number $a_3$ is configured to represent that the second bit-group and the first bit-group satisfy the following preset relationship: each bit in the first bit-group and the second bit-group has a value of 0 or 1 only, the second bit-group has two bits with different values only, there are $a_3$ consecutive second bit-groups immediately after the first bit-group, and a value of a first bit of the second bit-group is different from a value of a last bit of the first bit-group, $a_3$ being a positive integer;

a stroke repeat module, wherein the stroke repeat module is configured to represent that the second bit-group and the first bit-group satisfy the following preset relationship: the first content of the second bit-group is same as the first content of the first bit-group;

a bit increase module, wherein the bit increase module corresponding to a fourth preset number $a_4$ is configured to represent that the second bit-group and the first bit-group satisfy the following preset relationship: the second bit-group has one bit only, there are $a_4$ consecutive second bit-groups immediately after the first bit-group, and a value of the second bit-group is same as the value of the last bit of the first bit-group, $a_4$ being a positive integer; or

a bit decrease module, wherein the bit decrease module corresponding to a fifth preset number as is configured to represent that the second bit-group and the first bit-group satisfy the following preset relationship: as consecutive bits from back to front in the first bit-group are determined as the second bit-group, and the second bit-group in the first bit-group is removed, $a_5$ being a positive integer and $a_5$ being less than or equal to a total number of bits of the first bit-group.

In some embodiments, determining the molecular module corresponding to the at least one bit-group of the one or more bit-groups includes:

converting at least part of the information to be stored from a first system of numeration to a second system of numeration;

dividing the at least part of the information to be stored after conversion into one or more bit-groups; and

determining a molecular module corresponding to at least one bit-group of the one or more bit-groups obtained after conversion.

In some embodiments, the first system of numeration is binary, the second system of numeration is decimal, and converting the at least part of the information to be stored from the first system of numeration to the second system of numeration includes:
performing an iterative calculation according to $x_{i+1} = 2x_i + b_{i+1}$ to determine the at least part of the information to be stored in decimal, wherein $x_0$ is a preset initial value, $b_{i+1}$ is a value of $(i+1)$th bit from left to right in the at least part of the information to be stored in binary, $i$ being an integer greater than or equal to 0, and in a case that $(i+1)$ is equal to a total number of bits of the at least part of the information to be stored in binary, a corresponding $x_{i+1}$ is the at least part of the information to be stored in decimal.

In some embodiments, the first system of numeration is binary, the second system of numeration is decimal, and converting the at least part of the information to be stored from the first system of numeration to the second system of numeration includes:
calculating the at least part of the information to be stored

$$x = \sum_{n=0}^{i} 2^n * a_n$$

in decimal according to , wherein $a_n$ is a value of $(n+1)$th bit in the at least part of the information to be stored in binary, and $(i+1)$ is a total number of bits of the at least part of the information to be stored in binary.

In some embodiments, the first system of numeration is binary, the second system of numeration is an integer multiple of two base, and converting the at least part of the information to be stored from the first system of numeration to the second system of numeration includes:

determining whether the at least part of the information to be stored needs to be padded at a preset position according to the second system of numeration and a total number of bits of the at least part of the information to be stored,

if so, padding the at least part of the information to be stored according to the second system of numeration to obtain target information to be stored,

if not, directly taking the at least part of the information to be stored as the target information to be stored;

dividing the at least part of the information to be stored in binary into one or more pieces of sub-information according to the second system of numeration, wherein a number of bits of each piece of sub-information is an integer multiple of the second system of numeration with respect to the binary; and

converting each piece of sub-information in binary into a corresponding value in the second system of numeration to generate the at least part of the information to be stored in the second system of numeration.

In some embodiments, a relative position of the determined molecular module in the composition is consistent with a relative position of the corresponding bit-group in the information to be stored.

In some embodiments, generating the composition based on the determined molecular module such that the composition corresponds to the information to be stored includes:

obtaining a unit module corresponding to the determined molecular module, wherein each molecular module is synthesized from one or more unit modules; and

synthesizing the obtained unit module to generate the composition corresponding to the information to be stored.

In some embodiments, the determined molecular module includes at least one of a DNA fragment or an RNA fragment, and the unit module includes a nucleotide,

synthesizing the obtained unit module to generate the composition corresponding to the information to be stored includes:

synthesizing the composition directly starting from the obtained nucleotide, wherein at least a fragment of the composition is consistent with the determined molecular module.

In some embodiments, generating the composition based on the determined molecular module such that the composition corresponds to the information to be stored includes:

forming a terminal portion corresponding to a preset sequence at a connection end of the determined molecular module; and

mixing the molecular modules formed with the corresponding terminal portions to generate the composition corresponding to the information to be stored.

In some embodiments, a molecular fragment as the terminal portion is part of the corresponding molecular module; or

the molecular fragment as the terminal portion is added to the corresponding molecular module after the molecular module is determined.

In some embodiments, mixing the molecular modules formed with the corresponding terminal portions to generate the composition corresponding to the information to be stored includes at least one of:

combining the determined molecular modules in the preset sequence by using a ligase;

combining the determined molecular modules in the preset sequence by using linkers disposed at ends of the molecular modules; or

combining the determined molecular modules in the preset sequence by using a polymerase chain reaction.

In some embodiments, the molecular module includes at least one of: a deoxyribonucleic acid, a ribonucleic acid, a non-natural nucleotides, a modified nucleotides, an artificially synthetic nucleotide, a peptide, an organic polymer, an organic small molecule, a carbon nanomaterial, an inorganic substance, or spaced molecular fragments.

In some embodiments, various molecular modules are distinguished by at least one of: a sequence distribution, a sequence length, a secondary structure, a crystalline or amorphous nature or a morphology of the molecular modules.

In some embodiments, an edit distance between different molecular modules is greater than or equal to a preset distance threshold.

According to another aspect of the embodiments of the present disclosure, a method for storing information in a molecule is further provided, including:

obtaining information to be stored, wherein the information to be stored has one or more bit-groups, a position of each bit-group in the information to be stored is represented by a first address, a value of each bit-group is represented by a first content, and each bit-group has one or more bits;

determining one or more target bit-groups according to distribution of each kind of first content in the information to be stored;

determining a molecular module corresponding to each target bit-group of the one or more target bit-groups, wherein the molecular module includes a third molecular module, and the third molecular module is configured to represent the first address of a corresponding target bit-group; and

generating a composition based on the determined molecular module such that the composition corresponds to the information to be stored.

In some embodiments, obtaining the information to be stored includes:

dividing initial information to be stored to generate one or more pieces of information to be stored, wherein a number of bits of each piece of information to be stored is less than a number of bits of the initial information to be stored, and the number of bits of each piece of information to be stored is equal or not equal to each other; or

combining a plurality of pieces of initial information to be stored to generate the information to be stored, wherein the number of bits of the information to be stored is greater than the number of bits of each piece of initial information to be stored.

In some embodiments, determining the one or more target bit-groups according to the distribution of each kind of first content in the information to be stored includes:

counting an occurrence frequency of each kind of first content in the information to be stored, and determining a most frequent content with a highest occurrence frequency; and
determining a bit-group with a value other than the most frequent content as the target bit-group.

In some embodiments, the third molecular module includes one or more sub-molecule modules, and determining the molecular module corresponding to each target bit-group of the one or more target bit-groups includes:

dividing the first address of the target bit-group into one or more address bit-groups, wherein a position of each address bit-group in the first address of the target bit-group is represented by a second address, a value of each address bit-group is represented by a second content, and each address bit-group has one or more bits;
determining a sub-molecule module corresponding to at least one address bit-group of the one or more address bit-groups, wherein the sub-molecule module includes a first sub-molecule module, and the first sub-molecule module is configured to represent both the second address and the second content of a corresponding address bit-group; and
determining the third molecular module based on a combination of the determined sub-molecule module.

In some embodiments, determining the sub-molecule module corresponding to the at least one address bit-group of the one or more address bit-groups includes:

determining if a second address bit-group and a first address bit-group of the one or more address bit-groups satisfy a preset relationship, wherein the second address of the second address bit-group is different from the second address of the first address bit-group; and
in a case that the second address bit-group and the first address bit-group satisfy the preset relationship, determining the sub-molecule module corresponding to the second address bit-group, wherein the sub-molecule module includes a second sub-molecule module, and the second sub-molecule module is configured to represent the preset relationship between the corresponding second address bit-group and the first address bit-group.

In some embodiments, the second sub-molecule module includes:
a stroke increment module, wherein the stroke increment module corresponding to a sixth preset number $a_6$ is configured to represent that the second address bit-group of the second bit-group and the first address bit-group of the first bit-group satisfy the following preset relationship: a sum of the first address bit-group and $a_6$ is the second address bit-group, and the first content of the second bit-group is same as the first content of the first bit-group.

In some embodiments, the molecular module further includes a fourth molecular module, and the fourth molecular module is configured to represent the first content of the corresponding target bit-group.

In some embodiments, determining the molecular module corresponding to each target bit-group of the one or more target bit-groups includes:

converting at least part of the information to be stored from a first system of numeration to a second system of numeration;
dividing the at least part of the information to be stored after conversion into one or more bit-groups; and
determining a molecular module corresponding to at least one bit-group of the one or more bit-groups obtained after conversion.

In some embodiments, the first system of numeration is binary, the second system of numeration is decimal, and converting the at least part of the information to be stored from the first system of numeration to the second system of numeration includes:
performing an iterative calculation according to $x_{i+1} = 2x_i + b_{i+1}$ to determine the at least part of the information to be stored in decimal, wherein $x_0$ is a preset initial value, $b_{i+1}$ is a value of $(i+1)$th bit from left to right in the at least part of the information to be stored in binary, $i$ being an integer greater than or equal to 0, and in a case that $(i+1)$ is equal to a total number of bits of the at least part of the information to be stored in binary, a corresponding $x_{i+1}$ is the at least part of the information to be stored in decimal.

In some embodiments, the first system of numeration is binary, the second system of numeration is decimal, and converting the at least part of the information to be stored from the first system of numeration to the second system of numeration includes:
calculating the at least part of the information to be stored in decimal according to $x = \sum_{n=0}^{i} 2^n * a_n$, wherein $a_n$ is a value of $(n+1)$th bit in the at least part of the information to be stored in binary, and $(i+1)$ is a total number of bits of the at least part of the information to be stored in binary.

In some embodiments, the first system of numeration is binary, the second system of numeration is an integer multiple of two base, and converting the at least part of the information to be stored from the first system of numeration to the second system of numeration includes:

determining whether the at least part of the information to be stored needs to be padded at a preset position according to the second system of numeration and a total number of bits of the at least part of the information to be stored,

if so, padding the at least part of the information to be stored according to the second system of numeration to obtain target information to be stored,

if not, directly taking the at least part of the information to be stored as the target information to be stored;

dividing the at least part of the information to be stored in binary into one or more pieces of sub-information according to the second system of numeration, wherein a number of bits of each piece of sub-information is an integer multiple of the second system of numeration with respect to the binary; and converting each piece of sub-information in binary into a corresponding value in the second system of numeration to generate the at least part of the information to be stored in the second system of numeration.

In some embodiments, a relative position of the determined molecular module in the composition is consistent with a relative position of the corresponding bit-group in the information to be stored.

In some embodiments, generating the composition based on the determined molecular module such that the composition corresponds to the information to be stored includes:

obtaining a unit module corresponding to the determined molecular module, wherein each molecular module is synthesized from one or more unit modules; and

synthesizing the obtained unit module to generate the composition corresponding to the information to be stored.

In some embodiments, the determined molecular module includes at least one of a DNA fragment or an RNA fragment, and the unit module includes a nucleotide,

synthesizing the obtained unit module to generate the composition corresponding to the information to be stored includes:

synthesizing the composition directly starting from the obtained nucleotide, wherein at least a fragment of the composition is consistent with the determined molecular module.

In some embodiments, generating the composition based on the determined molecular module such that the composition corresponds to the information to be stored includes:

forming a terminal portion corresponding to a preset sequence at a connection end of the determined molecular module; and

mixing the molecular modules formed with the corresponding terminal portions to generate the composition corresponding to the information to be stored.

In some embodiments, a molecular fragment as the terminal portion is part of the corresponding molecular module; or

the molecular fragment as the terminal portion is added to the corresponding molecular module after the molecular module is determined.

In some embodiments, mixing the molecular modules formed with the corresponding terminal portions to generate the composition corresponding to the information to be stored includes at least one of:

combining the determined molecular modules in the preset sequence by using a ligase;

combining the determined molecular modules in the preset sequence by using linkers disposed at ends of the molecular modules; or

combining the determined molecular modules in the preset sequence by using a polymerase chain reaction.

In some embodiments, the molecular module includes at least one of: a deoxyribonucleic acid, a ribonucleic acid, a non-natural nucleotides, a modified nucleotides, an artificially synthetic nucleotide, a peptide, an organic polymer, an organic small molecule, a carbon nanomaterial, an inorganic substance, or spaced molecular fragments.

In some embodiments, various molecular modules are distinguished by at least one of: a sequence distribution, a sequence length, a secondary structure, a crystalline or amorphous nature or a morphology of the molecular modules.

In some embodiments, an edit distance between different molecular modules is greater than or equal to a preset distance threshold.

According to yet another aspect of the embodiments of the present disclosure, a device for storing information in a molecule is provided, including:

a memory having instructions stored thereon; and

a processor coupled to the memory, wherein the instructions, when executed by the processor, implement the above-mentioned method for storing information in a molecule.

According to still another aspect of the embodiments of the present disclosure, a system for storing information in a molecule is provided, including:

an obtaining unit configured to obtain information to be stored, wherein the information to be stored has one or more bit-groups, a position of each bit-group in the information to be stored is represented by a first address, a value of each bit-group is represented by a first content, and each bit-group has one or more bits;

a coding unit configured to determine a molecular module corresponding to at least one bit-group of the one or more bit-groups, wherein the molecular module includes a first molecular module, and the first molecular module is configured to represent both the first address and the first content of a corresponding bit-group; and

a writer unit configured to generate a composition based on the determined molecular module such that the composition corresponds to the information to be stored.

According to another aspect of the embodiments of the present disclosure, a system for storing information in a molecule is provided, including:

an obtaining unit configured to obtain information to be stored, wherein the information to be stored has one or more bit-groups, a position of each bit-group in the information to be stored is represented by a first address, a value of each bit-group is represented by a first content, and each bit-group has one or more bits;

a coding unit configured to determine one or more target bit-groups according to distribution of each kind of first content in the information to be stored; and to determine a molecular module corresponding to each target bit-group of the one or more target bit-groups, wherein the molecular module includes a third molecular module, and the third molecular module is configured to represent the first address of a corresponding target bit-group; and

a writer unit configured to generate a composition based on the determined molecular module such that the composition corresponds to the information to be stored.

According to yet another aspect of the embodiments of the present disclosure, a computer-readable storage medium is provided, the computer-readable storage medium having instructions stored thereon, wherein the instructions, when executed by a processor, implement the above-mentioned method for storing information in a molecule.

According to still another aspect of the embodiments of the present disclosure, a computer program product is provided, the computer program product including instructions that, when executed by a processor, implement the above-mentioned method for storing information in a molecule.

In the embodiments of the present disclosure, by converting a mapping relationship between the information to be stored and the molecular module into a mapping relationship between the data set extracted according to the information to be stored and the molecular module, the direct correspondence in the current molecular storage encoding between the information to be stored in binary and the molecular module is changed, the representation of part of the information may be compressed or defaulted according to the characteristic of the information to be stored, or one molecular module may be adopted to represent multiple types of information instead of only a single information, thereby achieving the massive reduction in the number of required molecular modules, increasing the encoding efficiency, improving the write speed, and reducing the cost of molecular storage.

The technical solutions of the present disclosure will be further described in detail below through the accompanying drawings and embodiments.

## BRIEF DESCRIPTION OF THE DRAWINGS

In order to illustrate the technical solutions in the embodiments of the present disclosure or in the prior art more clearly, the drawings required to be used in the description of the embodiments or the prior art will be briefly introduced below. Obviously, the drawings in the following description are only some embodiments of the present disclosure. For those of ordinary skill in the art, other drawings can be obtained according to these drawings without creative efforts.

Fig. 1 is a schematic flowchart of a method for storing information in a molecule according to an exemplary embodiment of the present disclosure;
Fig. 2(a) is a schematic diagram of the first molecular modules and a schematic diagram of using the first molecular modules to represent the information to be stored "1010" according to a specific example of the present disclosure;
Fig. 2(b) is a schematic diagram of the first molecular modules according to another specific example of the present disclosure;
Fig. 3(a) is a schematic diagram of the second molecular modules according to some specific examples in the present disclosure;
Fig. 3(b) is a schematic diagram of using the second molecular modules to represent a part of the information to be stored "101110" according to some specific examples of the present disclosure;
Fig. 3(c) is a schematic diagram of using the second molecular modules to represent the information to be stored "10101" according to a specific example of the present disclosure;
Fig. 4(a) is a schematic diagram of using the second molecular modules to represent a part of the information to be stored "0111001110" according to a specific example of the present disclosure;

Fig. 4(b) is a schematic diagram of using the second molecular modules to represent a part of the information to be stored "01110011110" according to a specific example of the present disclosure;

Fig. 4(c) is a schematic diagram of using the second molecular modules to represent a part of the information to be stored "011100110" according to a specific example of the present disclosure;

Fig. 5 is a schematic flowchart of a method for storing information in a molecule according to another exemplary embodiment of the present disclosure;

Fig. 6(a) is a schematic diagram of the information to be stored according to a specific example of the present disclosure;

Fig. 6(b) is a schematic diagram of using the third molecular modules to represent the information to be stored in Fig. 6(a);

Fig. 7(a) is a schematic diagram of the second sub-molecule modules according to a specific example of the present disclosure;

Fig. 7(b) is a schematic diagram of using the second sub-molecule modules to represent the information to be stored "0110010001" according to a specific example of the present disclosure;

Fig. 7(c) is a schematic diagram of using the second sub-molecule modules to represent the information to be stored "0111010001" according to a specific example of the present disclosure;

Fig. 8(a) is a schematic diagram of the representation of the information to be stored "1001101010" before conversion of the system of numeration according to a specific example of the present disclosure;

Fig. 8(b) is a schematic diagram of the representation of the information to be stored "1001101010" after conversion of the system of numeration according to a specific example of the present disclosure;

Fig. 9 shows a schematic diagram of combining the molecular modules according to a first specific example of the present disclosure;

Fig. 10 shows a schematic diagram of combining the molecular modules according to a second specific example of the present disclosure;

Fig. 11 shows a schematic diagram of combining the molecular modules according to a third specific example of the present disclosure;

Fig. 12 shows a schematic diagram of combining the molecular modules according to a fourth specific example of the present disclosure;

Fig. 13 shows a schematic diagram of combining the molecular modules according to a fifth specific example of the present disclosure;

Fig. 14 shows a schematic diagram of combining the molecular modules according to a fifth specific example of the present disclosure;

Fig. 15 shows a schematic diagram of combining the molecular modules according to a sixth specific example of the present disclosure;

Fig. 16 shows a schematic diagram of employing the chemical method or the enzymatic method to generate the composition according to a specific example of the present disclosure;

Fig. 17 is a schematic diagram of a device for storing information in a molecule according to an exemplary embodiment of the present disclosure;

Fig. 18 is a schematic diagram of a system for storing information in a molecule according to an exemplary embodiment of the present disclosure.

## DETAILED DESCRIPTION

The technical solutions in the embodiments of the present disclosure will be clearly and completely described below in conjunction with the accompanying drawings in the embodiments of the present disclosure. Obviously, the described embodiments are only some of the embodiments of the present disclosure, rather than all of the embodiments. Based on the embodiments in the present disclosure, all other embodiments obtained by those of ordinary skill in the art without making creative efforts fall within the scope of protection of the present disclosure.

The relative arrangement of components and steps, numerical expressions, and numerical values set forth in these embodiments do not limit the scope of the present disclosure unless otherwise specifically stated.

At the same time, it should be understood that, for convenience of description, the dimensions of various parts shown in the drawings are not drawn according to actual proportional relationships.

Techniques, methods and devices known to those of ordinary skill in the relevant art may not be discussed in detail, but where appropriate, such techniques, methods and devices should be considered as a part of the specification.

In all examples shown and discussed herein, any specific values are to be construed as illustrative only and not as limiting. Accordingly, other examples of the exemplary embodiments may have different values.

It should be noted that similar reference numerals and letters refer to similar items in the following figures, so that once an item is defined in one figure, it does not need further discussion in subsequent figures.

In addition, in the description of the present disclosure, the terms "first", "second", "third", and the like are used for descriptive purposes only and shall not be understood as indicating or implying relative importance and order. Similarly, although operations are depicted in a specific order in the drawings, this should not be understood as requiring that such operations be performed in the specific order shown, or in sequential order, or that all illustrated operations be performed to achieve desirable results. In some cases, multitasking and parallel processing may be advantageous.

In the molecular storage technology, for each bit in the information to be stored, different molecular modules may be used for representing its position and value respectively, and the complete information to be stored may be represented by synthesizing or combining these molecular modules. It can be understood that as the number of bits of the information to be stored increases, the types and the number of the molecular modules required also increase significantly, which results in the need to consume a large number of molecular modules to store the information, the speed of writing the information into the molecule produced by combining the molecular modules being very low, thereby resulting in a significant increase in various costs of the molecule storage.

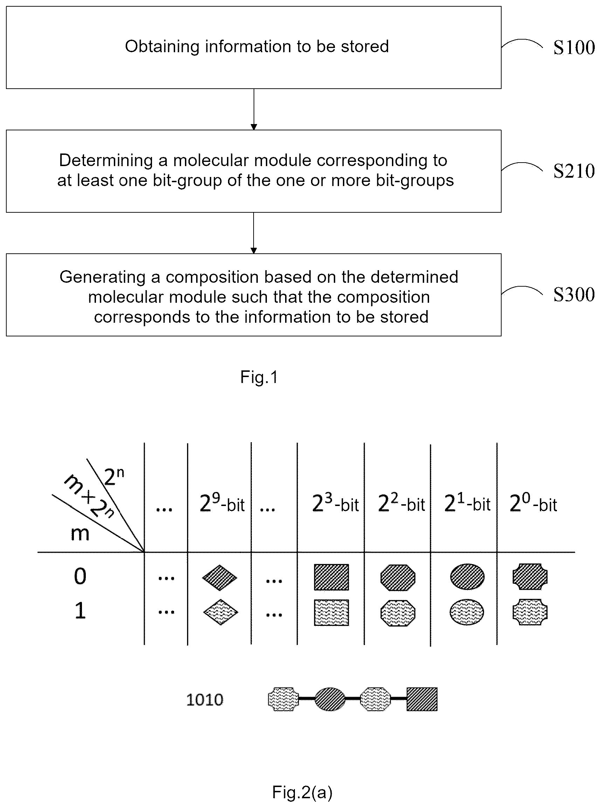

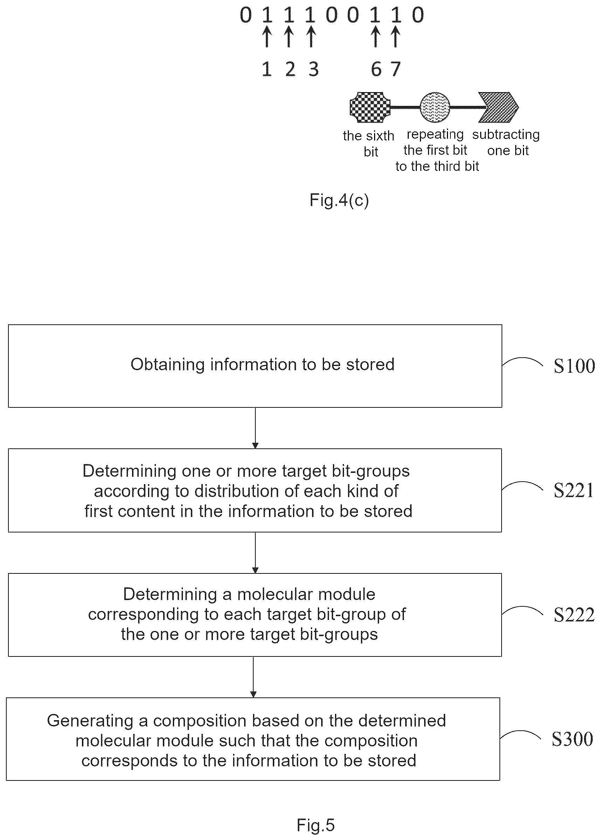

In order to solve the above problem, the present disclosure proposes a method for storing information in a molecule. In an exemplary embodiment of the present disclosure, as shown in Figs. 1 and 5, the method for storing the information in the molecule may include:
Step S100, obtaining information to be stored.

In production and life, there may be various forms of information, such as text information, picture information, audio information, video information, or the like. In order to facilitate the storage of these pieces of information, the information may be first converted into the information in binary encoding form, for example, by employing information technology. In the following, unless otherwise illustrated, the technical solutions of the present disclosure will be set forth in detail by taking the information to be stored being the binary information as an example. However, it can be understood that the information to be stored may be the information in other bases as desired, which is not limited here.

In addition, in some embodiments, some processing may be performed on the initial information to be stored in advance to simplify the subsequent storage steps. For example, a longer piece of initial information to be stored may be divided into multiple pieces of shorter information to be stored in advance. In a specific example, the initial information to be stored may be divided by a manner of fixed-length division, that is, the number of bits in each piece of divided information to be stored is equal to each other. Alternatively, in another specific example, the initial information to be stored may be divided by a manner of variable-length division, that is, each piece of divided information to be stored may have different number of bits.

Alternatively, in some embodiments, some pieces of initial information to be stored may be combined in advance, so that fewer compositions are used for representing a plurality of pieces of initial information to be stored. For example, for a plurality of pieces of initial information to be stored, a corresponding number of compositions are generally required to represent these pieces of initial information to be stored. Although the structure of each composition may be relatively simple, it also brings about the reduction in efficiency. In order to solve the above problem, a plurality of pieces of initial information to be stored may be merged into one piece of information to be stored, and one composition is used for representing this piece of information to be stored. As such, even though the structure of the composition may become more complex, the time required to synthesize one composition is usually shorter than that required to synthesize a plurality of compositions, thus helping to increase the efficiency. It can be understood that a plurality of pieces of initial information to be stored may be synthesized in various ways as desired, as long as the initial information to be stored may be uniquely derived according to the final composition.

Here, the information to be stored may have one or more bit-groups, and each bit-group may have one or more bits, wherein a position of each bit-group in the information to be stored may be represented by a first address, and a value of each bit-group may be represented by a first content. In some embodiments, the total number of bits in each divided bit-group in the information to be stored may be equal to each other. In some other embodiments, the total number of bits in each divided bit-group in the information to be stored may alternatively be unequal as desired.

For example, taking the information to be stored being the binary information "1010" as an example, this piece of information to be stored may be divided into four bit-groups, each bit-group has only one bit, the first address of each bit-group from left to right may be represented as "0", "1", "2" and "3" respectively (unless otherwise stated, the positions will be numbered starting from "0" herein), and accordingly, the first content of each bit-group may be "1", "0", "1" and "0" respectively. Alternatively, this piece of information to be stored may be divided into two bit-groups, each bit-group has two bits, the first address of each bit-group from left to right may be represented as "0" and "1" respectively, and the first content of each bit-group may be "10" and "10" respectively. Alternatively, this piece of information to be stored may be divided into one bit-group with four bits, the first address of this bit-group may be represented as "0", and the first content of this bit-group is "1010". In some embodiments, the specific way of dividing the bit-group may be determined according to the characteristic of the information to be stored and the types of molecular modules that may be used, so as to improve the coding efficiency of the molecular storage, to reduce the number of required molecular modules, to speed up writing, to reduce the storage cost, and the like.

Returning to Fig. 1, in an exemplary embodiment of the present disclosure, the method for storing the information in the molecule may further include:
Step S210, determining a molecular module corresponding to at least one bit-group of the one or more bit-groups.

The molecule module may include a first molecule module, and the first molecule module may be configured to represent both the first address and the first content of a corresponding bit-group. As such, by using one same molecule module to represent both the first address and the first content of one corresponding bit-group, compared to using two different molecule modules to represent the first address and the first content of the bit-group respectively, the number of the molecular modules required to represent the bit-group may be effectively reduced, thereby reducing the number of the molecular modules required to represent the entire information to be stored. Each bit-group here may have only one bit, as described in the specific example below. However, it can be understood that each bit-group may have two or more bits, and the corresponding molecular modules are used for representing the bit-groups with various first contents and first addresses, which is not limited here.

In a specific example, various molecular modules corresponding to the first contents and the first addresses of each bit in the binary information are listed in the table of Fig. 2(a), and the patterns with different shapes or fillings in the table are used for representing different molecular modules (i.e., unless otherwise stated, as long as either attributes (shape and filling) of two patterns used for representing two molecular modules are different, then the two molecular modules are different herein). In the table, $m$ represents the first content of each bit-group (bit), and its value may be 0 or 1; $n$ represents the first address of each bit-group (bit), for example, $n$ represents that the corresponding bit-group (bit) is a bit with the number of $n$ from left to right in the information to be stored, or is the $2^n$-bit in the binary information.

Based on the table in Fig. 2(a), when representing the information to be stored "1010" in binary, the following molecule modules in the table may be selected: the molecule module that represents the first address of "$2^\circ$" bit and the first content of " 1", the molecule module that represents the first address of "$2^1$" bit and the first content of "0", the molecule module that represents the first address of "$2^2$" bit and the first content of " 1", and the molecule module that represents the first address of "$2^3$" bit and the first content of "0", then these molecule modules are connected in a certain order (for example, in the arrangement order of each bit-group (bit) in the information to be stored), so that "1010" is stored in the corresponding molecule, as shown in the diagram below the table in Fig. 2(a).

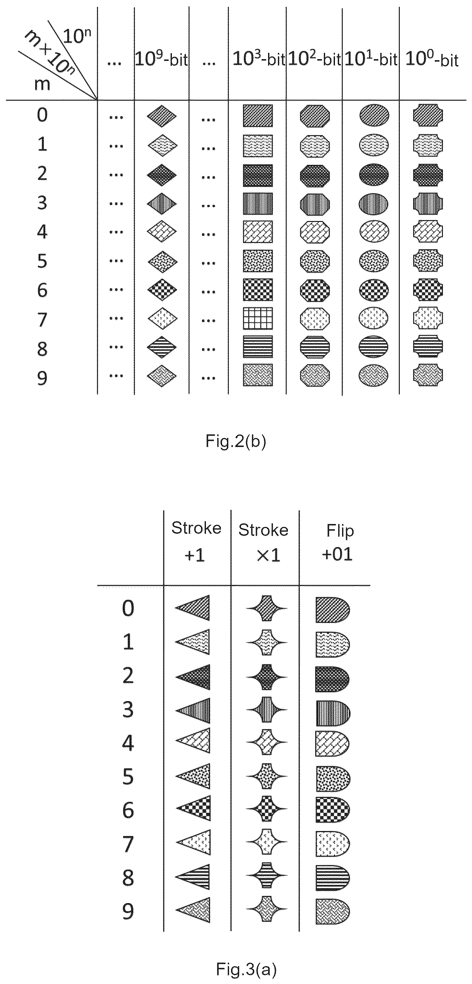

In another specific example, the molecule modules corresponding to each bit in the decimal information may be provided. When the information to be stored is the decimal information or the information to be stored is converted into the decimal information, it may be represented by the molecular modules shown in Fig. 2(b). In the table, $m$ represents the first content of each bit-group (bit), and its value may be 0 to 9; $n$ represents the first address of each bit-group (bit), for example, $n$ represents that the corresponding bit-group (bit) is a bit with the number of $n$ from left to right in the decimal information, or is the $10^n$-bit in the decimal information. A similar manner as described above with reference to Fig. 2(a) may be used for determining the molecular module corresponding to each bit in the decimal information, and the determined molecular modules may be combined to store the information in the molecule, which will not be described again here. Compared with the binary-based correspondence shown in Fig. 2(a), the decimal-based correspondence shown in Fig. 2(b) may use fewer molecular modules to represent the information to be stored. For example, from the $10^0$-bit to the $10^9$-bit, a total of 100 molecular modules may be utilized to represent up to $10^{10}$ bits of information, or approximately 1 gigabyte (GB) of information.

It can be understood that in some other embodiments, a corresponding molecular module library may be established, and accessing and combination of molecular modules may be employed to represent an arbitrary k-base information ($\sum m * k^n$), similar to that mentioned above, wherein $m$ represents the first content of each bit, and $n$ represents the first address of each bit in the information to be stored.

In some embodiments, determining the molecular module corresponding to the at least one bit-group of the one or more bit-groups may include: determining the first molecular module corresponding to each bit-group of the one or more bit-groups.

In this case, in the molecule used for representing the information to be stored, the number of the molecule modules may be consistent with the number of the bit-groups (bits) divided from the information to be stored. For example, in the molecule corresponding to the information to be stored "1010" as shown in Fig. 2(a), each bit-group (bit) has its corresponding molecular module combined in the entire molecule.

However, considering that under the normal circumstance, the information to be stored may have a plurality of bit-groups, and the values of the plurality of bit-groups may contain at least two kinds of first contents, if the first address and the first content of each bit-group need to be represented by a corresponding molecular module, the result may be that the number of required molecular modules is still large. In order to further reduce the number of required molecular modules, in some other embodiments, determining the molecular module corresponding to the at least one bit-group of the one or more bit-groups may include: determining that a bit-group with a value of one kind of first content does not correspond to any molecule module, and determining the first molecule module corresponding to at least one bit-group of other bit-group with a value of other kind of first content.

In other words, one kind of first content of multiple different kinds of first contents may be represented by default, that is, this kind of first content does not correspond to any molecular module, and the first molecular module is used for representing at least one bit-group with other kind of first content. When restoring the information stored in the molecule, the corresponding bit-group with the first content that was defaulted during storage may be supplemented, so as to restore the entire information to be stored.

In a specific example, in the case of binary information, only the bit-group (bit) with the first content of "0" may be represented, while the bit-group (bit) with the first content of "1" may be defaulted; alternatively, only the bit-group (bit) with the first content of "1" may be represented, while the bit-group (bit) with the first content of "0" may be defaulted. For example, according to the occurrence frequencies of the first contents "0" and "1", the bit-group (bit) with which first content to be defaulted may be determined. In some embodiments, the bit-group (bit) with the first content having a higher occurrence frequency may be defaulted to reduce the number of molecular modules in the molecule for representing the information to be stored.

In another specific example, in the case of decimal information, the bit-group (bit) with the first content of "0" may be defaulted, and corresponding molecule modules may be used for representing the bit-groups (bits) with the first contents of "1" to "9" respectively. Similarly, the bit-group (bit) with which first content to be defaulted may be determined according to the occurrence frequencies of various first contents. For example, the bit-group (bit) with the first content having the highest occurrence frequency is defaulted to reduce the number of molecular modules in the molecule for representing the information to be stored. It should be noted that when the first contents with the highest occurrence frequency include two or more first contents, any one of them may be selected to be defaulted.

Considering that in the practical application, the information to be stored usually has a large number of bit-groups (bits), if respective molecular modules corresponding to the bit-groups (bits) are directly combined together, the resulting molecule after combination is usually very large. Therefore, a corresponding bit-group may be represented according to a certain specific relationship between different bit-groups, so as to further reduce the number of molecular modules in the molecule for representing the information to be stored, to increase the writing speed, and to reduce the storage cost. In some embodiments, determining the molecular module corresponding to the at least one bit-group of the one or more bit-groups may include: determining if a second bit-group and a first bit-group of the one or more bit-groups satisfy a preset relationship, wherein the first address of the second bit-group is different from the first address of the first bit-group; and in a case that the second bit-group and the first bit-group satisfy the preset relationship, determining a molecular module corresponding to the second bit-group, wherein the molecular module includes a second molecular module, and the second molecular module is configured to represent the preset relationship between the corresponding second bit-group and the first bit-group.

Different preset relationships and corresponding molecular modules may be provided according to actual needs, which are not limited here. The following will take several preset relationships and corresponding second molecular modules as examples for illustration.

In some embodiments, the second molecule module may include a stroke incremental module, wherein the stroke incremental module corresponding to a first preset number $a_1$ is configured to represent a preset that the second bit-group and the first bit-group satisfy the following preset relationship: there are $a_1$ consecutive second bit-groups immediately after the first bit-group, and the first content of each second bit-group is same as the first content of the first bit-group, $a_1$ being a positive integer.

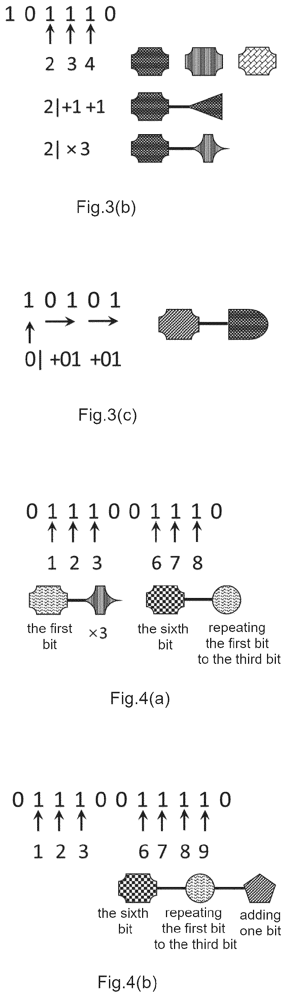

As shown in Fig. 3(a), the second column in the table lists the stroke incremental modules corresponding to various first preset numbers (as listed in the first column of the table). In a specific example, assuming that the information to be stored is "101110", if one molecular module is used for representing both the first address and the first content of one bit, then a total of three molecular modules are required to represent the bits numbered 3 to 5 from left to right in the information to be stored "101110", as shown by the first representation at the top in Fig. 3(b). However, considering that the first contents of the bits numbered 3 to 5 from left to right in the information to be stored "101110" are all "1", the stroke incremental module corresponding to the first preset number "2" shown in Fig. 3(a) may be used for representing the two consecutive bits after the bit with the first address numbered "3", as shown by the second representation in the middle in Fig. 3(b). The stroke incremental module corresponding to the first preset number "2" may be connected after the molecule module representing the bit with the first address numbered "3" in the information to be stored "101110", so as to indicate that immediately after this bit, there are two consecutive bits with the same first content as the first content of this bit. In the above specific example, each bit-group has only one bit. However, it can be understood that the stroke incremental module may be used in a bit-group with two or more bits for indicating that there are $a_1$ consecutive second bit-groups immediately after the first bit-group, and the first content of each second bit-group is the same as the first content of the first bit-group, while the first content here is formed by two or more bits.

In some embodiments, the second molecule module may include a stroke multiple module, wherein the stroke multiple module corresponding to a second preset number $a_2$ is configured to represent that the second bit-group and the first bit-group satisfy the following preset relationship: there are $(a_2-1)$ consecutive second bit-groups immediately after the first bit-group, and the first content of each second bit-group is same as the first content of the first bit-group, $a_2$ being a positive integer greater than 1.

As shown in Fig. 3(a), the third column in the table lists the stroke multiple modules corresponding to various second preset numbers (as listed in the first column of the table). In a specific example, assuming that the information to be stored is "101110", considering that the first contents of the bits with the first addresses numbered 3 to 5 from left to right of the information to be stored "101110" are all "1", thus they may be represented by using the stroke multiple module corresponding to the second preset number "3" shown in Fig. 3(a), as shown by the third representation at the bottom in Fig. 3(b). The stroke multiple module corresponding to the second preset number "3" may be connected after the molecule module representing the bit with the first address numbered "3" and the first content of "1" in the information to be stored "101110", so as to indicate that immediately after this bit, there are "3-1 = 2" consecutive bits with the same first content as that of this bit, or in other words, in the information to be stored, the first content with the first address numbered "3" will repeatedly appear for three times (including this bit itself). Similarly, in the above specific example, each bit-group has only one bit, however it can be understood that the stroke multiple module can be used in a bit-group with two or more bits for indicating that there are ($a_2$-1) consecutive second bit-groups immediately after the first bit-group, and the first content of each second bit-group is same as the first content of the first bit-group, while the first content here is formed by two or more bits.

In some embodiments, the second molecule module may include a stroke flip module, wherein the stroke flip module corresponding to a third preset number $a_3$ is configured to represent that the second bit-group and the first bit-group satisfy the following preset relationship: each bit in the first bit-group and the second bit-group has a value of 0 or 1 only, the second bit-group has two bits with different values only, there are $a_3$ consecutive second bit-groups immediately after the first bit-group, and a value of a first bit of the second bit-group is different from a value of a last bit of the first bit-group, $a_3$ being a positive integer.

As shown in Fig. 3(a), the fourth column in the table lists the stroke flip modules corresponding to various third preset numbers (as listed in the first column of the table). In a specific example, as shown in Fig. 3(c), assuming that the information to be stored is "10101", that is, immediately after the bit with the first address numbered "0" in the information to be stored, there are two consecutive such second bit-groups, the value of the first bit in the second bit-group is different from that of the last bit in the previous bit-group, and the values of the two bits in the second bit-group are different. Therefore, the stroke flip module corresponding to the third preset number "2" in Fig. 3(a) may be used to be connected after the first molecule module representing the bit with the first address numbered "0" and the first content of "1", so as to represent the information to be stored "10101".

In some embodiments, the second molecule module may include a stroke repeat module, wherein the stroke repeat module is configured to represent that the second bit-group and the first bit-group satisfy the following preset relationship: the first content of the second bit-group is same as the first content of the first bit-group.

In a specific example, as shown in Fig. 4(a), assuming that the information to be stored is "0111001110". Here, the bits with the first addresses of 1 to 3 in the information to be stored are all "1". The first contents of these three bits may be represented by a combination of the molecular module representing the first address of "1" and the first content of "1" and the stroke multiple module corresponding to the second preset number "3" in Fig. 3(a). In addition, the first contents of the bits with the first addresses of 6 to 8 are also all "1", which are same as those of the bits with the first addresses of 1 to 3. At this time, a combination of the molecular module representing the first address of 6 (which is used for indicating the starting position of repetition) and the stroke repeat module may be used for representing the bits with the first addresses of 6 to 8 in the information to be stored.

It can be understood that in different embodiments, different stroke repeat modules may be used for indicating the repetition of the first contents for different bits or bit-groups. In addition, the position of the repeated bit or bit-group indicated by the stroke repeat module in the information to be stored may be in front of the bit or bit-group represented by the stroke repeat module, or may be after the bit or bit-group represented by the stroke repeat module. In addition, the stroke repeat module can represent the repetition of various types of molecular modules. For example, it may represent not only the repetition of the first molecular module indicating both the first address and the first content of the bit or bit-group, but also the repetition of the second molecular module indicating the preset relationship between different bit-groups, or it may represent the repetition of other types of molecular modules that may exist and the like, which is not limited here. In addition, the bit-group represented by the stroke repeat module may have only one bit, or may have a plurality of bits, which is not limited here.

In some embodiments, the second molecular module may include a bit increase module and/or a bit decrease module. Here, the bit increase module and/or the bit decrease module may be used in combination with other molecular modules, for example, the stroke repeat module and the like.

The bit increase module corresponding to a fourth preset number $a_4$ is configured to represent that the second bit-group and the first bit-group satisfy the following preset relationship: the second bit-group has only one bit, there are $a_4$ consecutive second bit-groups immediately after the first bit-group, and a value of the second bit-group is same as the value of the last bit of the first bit-group, $a_4$ being a positive integer.

In a specific example, as shown in Fig. 4(b), assuming that the information to be stored is "01110011110", in which the first contents of the bits with the first addresses of 1 to 3 are all "1", and the first contents of the bits with the first addresses of 6 to 9 are also all "1". For the four bits with the first addresses of 6 to 9, on the basis of Fig. 4(a), the bit increase module corresponding to the fourth preset number "1" may be added after the stroke repeat module for representing that one more bit, which is same as the last bit of the previous bit-group, is added.

In addition, the bit decrease module corresponding to a fifth preset number as is configured to represent that the second bit-group and the first bit-group satisfy the following preset relationship: as consecutive bits from back to front in the first bit-group are determined as the second bit-group, and the second bit-group in the first bit-group is removed, as being a positive integer and as being less than or equal to a total number of bits of the first bit-group.

In a specific example, as shown in Fig. 4(c), assuming that the information to be stored is "011100110". Compared with the information to be stored "0111001110" in Fig. 4(a), the bit with the first address of "8" is removed. Therefore, on the basis of Fig. 4(a), the bit decrease module corresponding to the fifth preset number "1" may be added after the stroke repeat module for representing that the last bit of the previous first bit-group is subtracted.

In some embodiments, the information to be stored may be converted in system of numeration as desired, and the molecular module corresponding to at least one bit-group is determined based on the converted information to be stored. In particular, determining the molecular module corresponding to the at least one bit-group of the one or more bit-groups may include: converting at least part of the information to be stored from a first system of numeration to a second system of numeration; dividing the at least part of the information to be stored after conversion into one or more bit-groups; and determining a molecular module corresponding to at least one bit-group of the one or more bit-groups obtained after conversion. For example, the binary information to be stored may be converted to the decimal information to be stored or the like, to reduce the molecular modules required.

In a specific example, the conversion between the first system of numeration and the second system of numeration may be performed based on a common base conversion method. Here, the first system of numeration or the second system of numeration may be binary, octal, decimal, hexadecimal, sixty-four base, hundred base, or the like.

In particular, if the first system of numeration is binary and the second system of numeration is decimal, converting the at least part of the information to be stored from the first system of numeration to the second system of numeration may include: calculating the at least part of the information to be stored in decimal according to

$$x = \sum_{n=0}^{i} 2^n * a_n$$, wherein $a_n$ is a value of $(n+1)$

th bit in the at least part of the information to be stored in binary, and $(i+1)$ is a total number of bits of the at least part of the information to be stored in binary.

For example, for a piece of binary information to be stored $x(b) = a_i a_{i-1} ... a_1 a_0$, it may be converted into the decimal information to be stored $x(d)$ according to $x(d) = 2^0 * a_0 + 2^1 * a_1 + ... + 2^{i-1} * a_{i-1} + 2^i * a_i$. For example, in the above manner, the binary information to be stored "1001101010" may be converted into "618" in decimal.

In some other embodiments, if the first system of numeration is binary and the second system of numeration is an integer multiple of two base, converting the at least part of the information to be stored from the first system of numeration to the second system of numeration may include: determining whether the at least part of the information to be stored needs to be padded at a preset position according to the second system of numeration and the total number of bits of the at least part of the information to be stored; if so, padding the at least part of the information to be stored according to the second system of numeration to obtain target information to be stored, if not, directly taking the at least part of the information to be stored as the target information to be stored; dividing the at least part of the information to be stored in binary into one or more pieces of sub-information according to the second system of numeration, wherein the number of bits in each piece of sub-information is an integer multiple of the second system of numeration with respect to the binary; and converting each piece of sub-information in binary into a corresponding value in the second system of numeration to generate the at least part of the information to be stored in the second system of numeration.

For example, assuming that the first system of numeration is binary and the second system of numeration is octal, then for a piece of binary information to be stored $x(b) = a_i a_{i-1} ... a_1 a_0$, if its number of bits $(i+1)$ is an integer multiple of 3, $x(b)$ may be directly divided into several pieces of sub-information, each of which has three bits; or if the number of bits $(i+1)$ is not an integer multiple of 3, the binary information to be stored $x(b)$ may be padded with a corresponding number of bits at a preset position of the head, tail or middle thereof, so that the number of bits of the padded binary information to be stored is an integer multiple of 3, and then the padded information to be stored is divided into several pieces of sub-information, each of which has three bits. After that, each piece of sub-information is converted into a corresponding value from 0 to 7, so as to convert the binary information to be stored into the octal information to be stored $x(o)$. For example, in the above manner, by padding two bits of "0" to the head of the binary information to be stored "1001101010", it may be converted into "1152" in octal.

Assuming that the first system of numeration is binary and the second system of numeration is hexadecimal, then for a piece of binary information to be stored $x(b) = a_i a_{i-1} ... a_1 a_0$, if its number of bits $(i+1)$ is an integer multiple of 4, x(b) may be directly divided into several pieces of sub-information, each of which has four bits; or if the number of bits (i+1) is not an integer multiple of 4, the binary information to be stored x(b) may be padded with a corresponding number of bits at a preset position of the head, tail or middle thereof, so that the number of bits of the padded binary information to be stored is an integer multiple of 4, and then the padded information to be stored is divided into several pieces of sub-information, each of which has four bits. After that, each piece of sub-information is converted into a corresponding value from 0 to g respectively, so as to convert the binary information to be stored into the hexadecimal information to be stored x(h). For example, in the above manner, by padding two bits of "0" to the head of the binary information to be stored "1001101010", it may be converted into "26a" in hexadecimal.

Similarly, for the binary information to be stored "1001101010", it may be converted into the sixty-four base information to be stored "({9}{42})$_{64}$", or the hundred base information to be stored "({6}{18})$_{100}$", or the like.

In another specific example, other conversion method may be used for performing the conversion between different systems of numeration. For example, the information to be stored may be converted based on a one-to-one conversion relationship to reduce the number of molecular modules required during the storage process. In some embodiments, linear conversion method may be used for performing the conversion of the systems of numeration. For example, iterative calculation may be performed according to the following formula to convert at least a part of the information to be stored from binary to another system of numeration:

$$x_{i+1} = ax_i + b_{i+1},$$

wherein $a$ is a preset conversion coefficient, $x_0$ is a preset initial value, which may be determined as desired. In a specific example, the preset conversion coefficient $a = 2$ and the preset initial value $x_0 = 1$, so that the final converted $x_{i+1}$ may be made as small as possible to save the number of molecular modules required for storage. $b_{i+1}$ is a value of (i+1)th bit from left to right in the binary information (as mentioned above, i increases from 0, and i is an integer). When (i+1) is equal to the total number of bits of the binary information to be stored, the corresponding $x_{i-1}$ is the information to be stored in the target system of numeration. In a specific example, the target system of numeration may be decimal. Alternatively, after converting the binary information to be stored into decimal information to be stored based on the above formula, the decimal information to be stored may be further converted into information to be stored in another system of numeration based on the conversion relationship between decimal and the other system of numeration. In addition, the preset conversion coefficient and/or the preset initial value in the above formula may be changed as desired, which is not limited here.

For example, in a case converting the binary information to be stored "1001101010" into the decimal information to be stored, and the preset conversion coefficient being $a = 2$, the specific conversion process is as follows:

$$x_1 = 2x_0 + b_1 = 3,$$

$$x_2 = 2x_1 + b_2 = 6,$$

$$x_3 = 2x_2 + b_3 = 12,$$

$$x_4 = 2x_3 + b_4 = 25,$$

$$x_5 = 2x_4 + b_5 = 51,$$

$$x_6 = 2x_5 + b_6 = 102,$$

$$x_7 = 2x_6 + b_7 = 205,$$

$$x_8 = 2x_7 + b_8 = 410,$$

$$x_9 = 2x_8 + b_9 = 821,$$

$$x_{10} = 2x_9 + b_{10} = 1642.$$

That is, by using the above iterative calculation method, the binary information to be stored "1001101010" is converted into "1642" in decimal. Then, the method as described above may be used for determining one or more molecular modules corresponding to each bit-group in "1642", which will not be described again here.

In addition, in a case restoring the stored decimal information, it may be realized according to the restoration method corresponding to the conversion method. For example, based on the above conversion method, the restoring of "1642" may be performed in the following process:

$$(x_9, b_{10}) = ([x_{10}/a], x_{10} \bmod a) = (821, 0),$$

$$(x_8, b_9) = ([x_9/a], x_9 \bmod a) = (410, 1),$$

$$(x_7, b_8) = ([x_8/a], x_8 \bmod a) = (205, 0),$$

$$(x_6, b_7) = ([x_7/a], x_7 \bmod a) = (102, 1),$$

$$(x_5, b_6) = ([x_6/a], x_6 \bmod a) = (51, 0),$$

$$(x_4, b_5) = ([x_5/a], x_5 \bmod a) = (25, 1),$$

$$(x_3, b_4) = ([x_4/a], x_4 \bmod a) = (12, 1),$$

$$(x_2, b_3) = ([x_3/a], x_3 \bmod a) = (6, 0),$$

$$(x_1, b_2) = ([x_2/a], x_2 \bmod a) = (3, 0),$$

$$(x_0, b_1) = ([x_1/a], x_1 \bmod a) = (1, 1),$$

Finally, the restored binary information is "1001101010".

It can be understood that in some other embodiments, other methods may be used for performing the conversion between systems of numeration, as long as the information to be stored before and after conversion is in an one-to-one correspondence, and there is no limitation here. In addition, the conversion of systems of numeration is not limited between binary and decimal, but the systems of numeration before and after conversion may be determined as desired.

In another exemplary embodiment of the present disclosure, as shown in Fig. 5, a method for storing information in a molecule may include:

Step S221, determining one or more target bit-groups according to distribution of each kind of first content in the information to be stored.

In a specific example, determining the one or more target bit-groups according to the distribution of each kind of first content in the information to be stored may include: counting an occurrence frequency of each kind of first content in the information to be stored, and determining a most frequent content with a highest occurrence frequency; and determining a bit-group with a value other than the most frequent content as the target bit-group.

In this way, the bit-group with the first content having less occurrence frequency may be taken as the target bit-group as much as possible, and corresponding molecular modules are used for representing these target bit-groups in the subsequent steps, which saves the molecular modules consumed in the process of storing information, and also helps to increase the speed of writing the information to be stored into the molecule, thereby reducing the cost of molecule storage. It should be noted that if there are two different kinds of first contents in the information to be stored, and both of their occurrence frequencies are the highest, then the bit-group with only one kind of first content is selected as the defaulted bit-group (that is, which does not correspond to any molecule module), and the bit-group with the other kind of first content and other bit-groups with the first contents having lower occurrence frequencies are used as the target bit-groups, and the corresponding molecule modules are used in the subsequent steps to represent these target bit-groups.

Returning to Fig. 5, the method for storing information in the molecule may further include:

Step S222, determining a molecular module corresponding to each target bit-group of the one or more target bit-groups, wherein the molecular module includes a third molecular module, and the third molecular module is configured to represent the first address of a corresponding target bit-group.

In some embodiments, the third molecular module may be composed of one or more molecular modules. For example, in a specific example, each first address may be regarded as one piece of "information to be stored", one or more molecular modules corresponding to this first address may be determined based on the method described in Fig. 1, and the combination of the one or more molecular modules may be determined as the third molecular module.

In particular, the third molecule module for representing the first address of the target bit-group may include one or more first sub-molecule modules, and determining the molecular module corresponding to each target bit-group of the one or more target bit-groups includes: dividing the first address of the target bit-group into one or more address bit-groups, wherein a position of each address bit-group in the first address of the target bit-group is represented by a second address, a value of each address bit-group is represented by a second content, and each address bit-group has one or more bits; determining a sub-molecule module corresponding to at least one address bit-group of the one or more address bit-groups, wherein the sub-molecule module includes a first sub-molecule module, and the first sub-molecule module is configured to represent both the second address and the second content of a corresponding address bit-group; and determining the third molecular module according to a combination of the determined sub-molecule module. When a first address is regarded as a piece of "information to be stored", the first sub-molecule module may be considered to be equivalent to the "first molecular module" in the embodiment shown in Fig. 1. In addition, it can be understood that when representing the first address, the corresponding molecular module may be determined based on the preset relationship between different address bit-groups in the first address, that is, the various "second molecular modules" mentioned above may also be used for representing the first address.

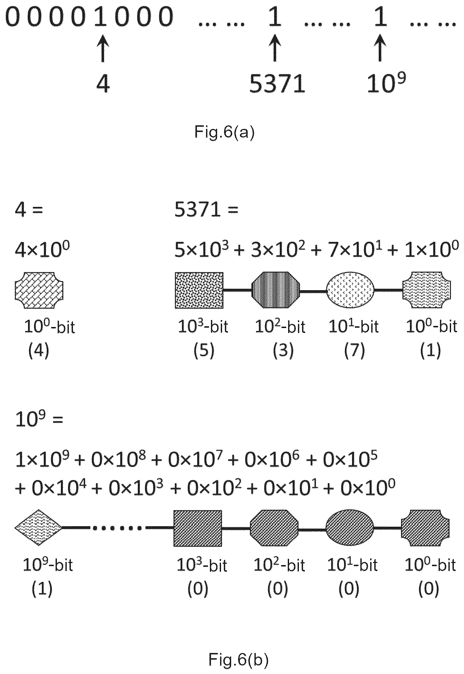

In a specific example, assuming that the information to be stored is as shown in Fig. 6(a), the bit-group (bit) with the first content "1" with the smallest occurrence frequency may be selected as the target bit-group, and the first address of the target bit-group may be stored. Here, the first addresses required to be stored include "4", "5371" and "10⁹". As shown in Fig. 6(b), these three addresses may be represented by using various molecular modules shown in Fig. 2(b) to form three corresponding molecular module chains. Moreover, since there are only two kinds of different first contents, as long as the first address of the bit-group with one kind of first content is determined, the first address of the bit with the other kind of first content may be determined, thus a molecular module corresponding to any kind of first content may not be provided.

In addition, in some embodiments, if the occurrence frequency of each of "0" and "1" in the information to be stored is 50%, then the bit-group with the first content of "0" may be taken as the target bit-group and its first address may be stored. Alternatively, the bit-group with the first content of "1" may be taken as the target bit-group, and its first address may be stored.

It can be understood that if the occurrence frequency of each of "0" and "1" in the information to be stored is 50%, then the number of the molecular modules saved will be limited by determining the target bit-group based on one of the first content of "0" and "1", and storing the first address of the target bit-group. Although the occurrence frequencies of "0" and "1" are the same, considering that the possibility that the occurrence frequencies of various combinations of "0" and "1" are the same concurrently is low, it may be considered to make each bit-group contain two or more bits, so as to find the bit-groups that appear less frequently, thereby reducing the number of molecular modules required as much as possible.

For example, in the binary information to be stored "100110001110", the occurrence frequencies of "0" and "1" are the same (50%), but the occurrence frequencies of the bit-group "10", the bit-group "01", the bit-group "00" and the bit-group "11" are not all the same, they are 50%, 16.67%, 16.67% and 16.67% respectively. At this time, the bit-groups with first contents of "01", "00" and "11" may be determined as the target bit-groups, and the first addresses of these target bit-groups may be stored. Simultaneously, since there are four different first contents in total, it is also necessary to provide the molecular modules corresponding to at least three kinds of first contents to distinguish the different first contents. Here, the molecule module may further include a fourth molecule module, and the fourth molecule module may be configured to represent the first content of a corresponding target bit-group. For example, the fourth molecular modules representing the first contents of "01", "00" and "11" may be respectively connected to at least one position of the front, middle and tail end of the molecular module representing the first address.

In this way, by dividing the information to be stored into a plurality of bit-groups, counting the occurrence frequency of each bit-group to determine the target bit-group, and determining the molecular module corresponding to the target bit-group, the consumption of the molecular modules may be further reduced, the speed of writing the information to be stored into the molecule may be improved, and the cost may be reduced. Here, the specific division method of the bit-groups in the information to be stored may be determined according to factors such as the characteristic of the information to be stored and the like, so as to reduce the required molecular modules as much as possible.

In addition, in some embodiments, after the target bit-group is determined, a certain conversion may be performed on the first address of the target bit-group to reduce the maximum value of the first address required to be represented. For example, for the information to be stored "1001110000110110" with a total of 16 bits, the first addresses of the bits with the value of "1" from left to right are "0", "3", "4", "5", "10", "11", "13", "14", respectively, the middle address of the information to be stored with 16 bits is "16/2 = 8", then the above first address sequence may be rewritten as "0", "3", "4", "5", "6", "5", "3", "2". That is, the value of the first address that is less than the middle address "8" is kept unchanged, and the value of the first address that is greater than the middle address "8" is converted into a difference between the number of the bits and the first address. In this way, the maximum value of the converted first address is 8, which can reduce the number of the molecular modules required for representing all the possible first addresses. Further, the idea of cyclic segmentation may be used for performing the similar operation as above on the first address sequence A: "0", "3", "4", "5" and the first address sequence B: "10", "11", "13", "14" respectively, so that the possible maximum value of the first address is further converted to "16/4 = 4". After conversion, a total of four first address sequences may be obtained, namely A1: "0", "3", "4", A2: "8-5 = 3", B1: "8-6 = 2", "8-5 = 3" and B2: "3", "2".

Furthermore, in some embodiments, determining the sub-molecule module corresponding to the at least one address bit-group of the one or more address bit-groups may include: determining if a second address bit-group and a first address bit-group of the one or more address bit-groups satisfy a preset relationship, wherein the second address of the second address bit-group is different from the second address of the first address bit-group; and in a case that the second address bit-group and the first address bit-group satisfy the preset relationship, determining the sub-molecule module corresponding to the second address bit-group, wherein the sub-molecule module includes a second sub-molecule module, and the second sub-molecule module is configured to represent the preset relationship between the corresponding second address bit-group and the first address bit-group.

In some embodiments, as mentioned above, if a first address is regarded as a piece of "information to be stored", then the "second sub-molecule module" here is equivalent to the "second molecule module" mentioned above.

Furthermore, in some embodiments, the second sub-molecule module may include a stroke increment module, wherein the stroke increment module corresponding to a sixth preset number $a_6$ is configured to represent that the second address bit-group of the second bit-group and the first address bit-group of the first bit-group satisfy the following preset relationship: a sum of the first address bit-group and $a_6$ is the second address bit-group, and the first content of the second bit-group is same as the first content of the first bit-group.

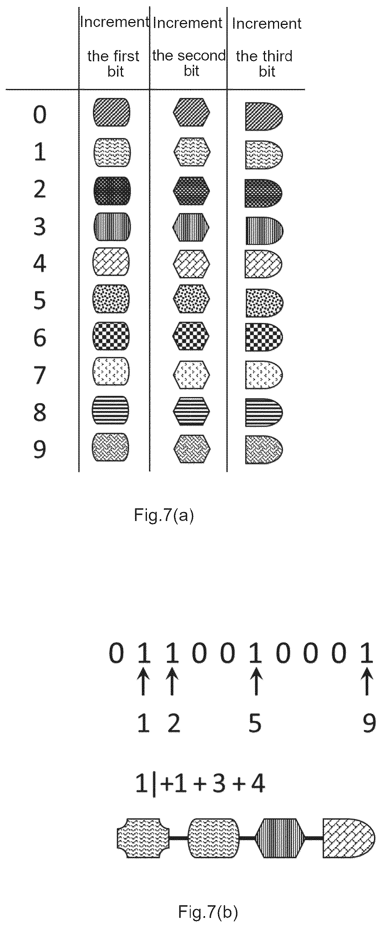

In a specific example, Fig. 7(a) shows a schematic diagram of various stroke increment modules, in which the first column represents the sixth preset number, the second column represents the increment of the second bit-group relative to the first bit-group, the third column represents the increment of the third bit-group relative to the second bit-group, and the fourth column represents the increment of the fourth bit-group relative to the third bit-group. As shown in Fig. 7(b), assuming that the information to be stored is "0110010001", it is determined that the bit-group with the first content of "1" is taken as the target bit-group, then the first address of the first target bit-group is "1", the first address of the second target bit-group is obtained by adding "1" to the first address of the first target bit-group, the first address of the third target bit-group is obtained by adding "3" to the first address of the second target bit-group, and the first address of the fourth target bit-group is obtained by adding "4" to the first address of the third target bit-group. Therefore, as shown in Fig. 7(b), the information to be stored "0110010001" may be represented by a combination of the molecule module representing the first target bit-group, the molecule module representing the second target bit-group with the first address increment of "1", the molecule module representing the third target bit-group with the first address increment of "3", and the molecule module representing the fourth target bit-group with the first address increment of "4" in Fig. 7(a).

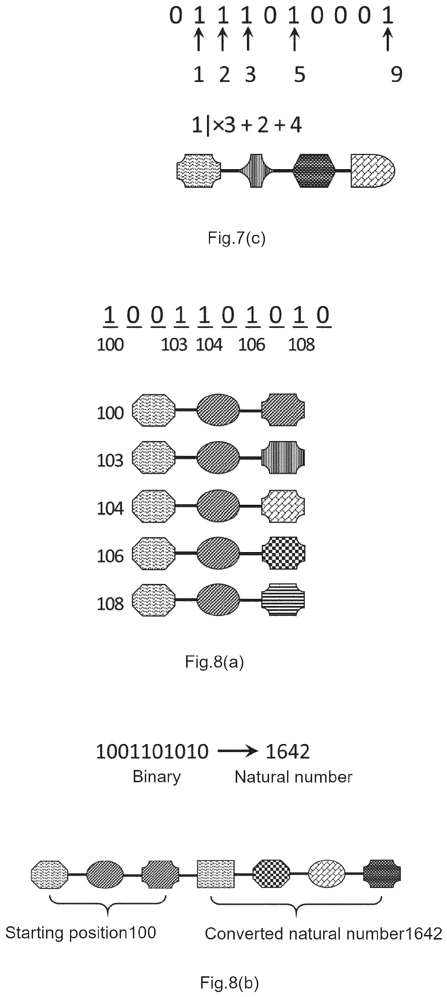

It can be understood that the various molecular modules described herein may be used in combination, as shown in Fig. 7(c). Alternatively, different molecular modules may be used for representing a same piece of information to be stored as desired, as shown in Fig. 3(b).

In some embodiments, the information to be stored may be represented by combining the first address of at least one target bit-group and the conversion of system of numeration on the corresponding information to be stored. Determining the molecular module corresponding to each target bit-group of the one or more target bit-groups may include: converting at least part of the information to be stored from a first system of numeration to a second system of numeration; dividing the at least part of the information to be stored after conversion into one or more bit-groups; and determining a molecular module corresponding to at least one bit-group of the one or more bit-groups obtained after conversion.

In a specific example, as shown in Figs. 8(a) and 8(b), assuming that a part of the information to be stored is "1001101010" (from the first address of 100 to the first address of 109). In the storage method shown in Fig. 8(a), five molecular module chains may be respectively used for representing the first addresses "100", "103", "104", "106" and "108" of the bits with the first content of "1" in the information to be stored. While in the storage method shown in Fig. 8(b), this part of the information to be stored may first be converted to decimal, and then the starting position (first address) "100" of this part of the information to be stored and the converted decimal value are stored to reduce the required molecule modules.

Furthermore, in some embodiments, the information to be stored with a larger number of bit-groups (bits) may be divided into several pieces of sub-information. For each piece of sub-information, the method described above may be used for determining the corresponding molecular module; or in other words, each piece of sub-information may be regarded as one piece of "information to be stored", and the molecular module corresponding to the piece of "information to be stored" may be determined using the method described above. Further, a certain molecular module may be additionally added to represent the molecular address of each piece of sub-information in the entire information to be stored to distinguish different pieces of sub-information. For example, 1024 molecule addresses may be used for dividing the information to be stored of 1 TB into multiple pieces of sub-information of 1 GB each, and each piece of sub-information may be represented utilizing the molecule modules as described above (in a specific example, for example, the molecule modules shown in Fig. 2(b)), and then combining with the added molecule module for representing the molecular address of each piece of sub-information, so as to determine the molecule corresponding to the entire information to be stored.

In some embodiments, a reference code may be provided. For example, the 1001st, 1002nd, and 1003rd bits of the information to be stored may take the 1000th bit as the reference, and may be represented as the 1st, 2nd, and 3rd bits respectively. As such, in a case that the molecular module is used for storage, the molecular module representing the reference code "1000" may be combined with the molecular modules representing the 1st, 2nd, and 3rd bits respectively to represent the corresponding information to be stored, which can further reduce the amount of usage of the molecular modules, increase the writing speed, and reduce the cost.

Returning to Figs. 1 and 5, a method for storing information in a molecule may further include:
Step S300, generating a composition based on the determined molecular module such that the composition corresponds to the information to be stored.

In some embodiments, the determined molecular modules may be directly obtained and combined in the preset sequence. In some other embodiments, considering that the molecular modules may be synthesized from more basic unit modules (for example, a DNA fragment as the molecular module may be synthesized from one or more nucleotides as unit modules), the types of unit modules themselves may usually be fewer, and the acquisition, transportation and storage of such unit modules may be more convenient, thus the unit modules corresponding to the various determined molecular modules may be obtained, instead of directly obtaining the molecular modules themselves, and synthesis may be performed starting from the unit modules to generate the composition corresponding to the information to be stored. Here, the relative position of the determined molecular module in the composition may be consistent with the relative position of the corresponding bit-group in the information to be stored.

In the embodiments of the present disclosure, the molecular module may include at least one of a deoxyribonucleic acid (DNA), a ribonucleic acid (RNA), a non-natural nucleotide, a modified nucleotide, an artificially synthetic nucleotides, a peptide, an organic polymer, an organic small molecule, a carbon nanomaterial, an inorganic substance, or spaced molecular fragments. When storing the information, the combination of different molecular modules is involved. These molecular modules may be combined together by a mode of action therebetween, such as a covalent bond, an ionic bond, a hydrogen bond, an intermolecular force, a hydrophobic force, a complementary base pairing or the like.

Among the above molecules, DNA (deoxyribonucleic acid) molecules are relatively suitable to serve as the molecular modules for molecular storage due to their properties. Firstly, the storage density of DNA molecules can theoretically reach more than $10^6$ to $10^7$ times that of the traditional storage media; secondly, DNA molecules have very strict base pairing principles; thirdly, DNA molecules are very stable and may be preserved under dry and low-temperature conditions for more than a thousand years.

Thus, DNA molecules may be preferably used for storing the information to reduce the cost of data storage operation and maintenance by orders of magnitude. In addition, DNA storage also has great advantages over the traditional storage methods in terms of carbon emission and energy consumption, data security, portability and the like.

In some embodiments, the molecular modules may be distinguished by at least one of a sequence distribution, a sequence length, a secondary structure, a crystalline or amorphous nature, or a morphology. For example, different sequences, different lengths, or different secondary structures of DNAs, RNAs, peptides, or organic polymers may be used for representing different values of different contents. In addition, different chemical forms, physical properties, crystalline or amorphous nature, and morphologies of DNAs, RNAs, peptides, organic polymers, organic small molecules, carbon nanomaterials, or inorganic substances may be used for representing different values of different contents.

Furthermore, the composition obtained by combining the molecular modules may be a mixture or a compound. For example, the composition may include a plurality of different DNA chains, which may respectively represent one or more pieces of information fragments to be stored in the information to be stored, and which may be mixed together to represent the complete information to be stored. Alternatively, DNA chains representing various information fragments to be stored may be further synthesized into a longer DNA chain to represent the complete information to be stored in a form of compound. Yet alternatively, synthesis may be performed starting from more basic nucleotides to generate the DNA chain corresponding to the information to be stored.

It should be understood that in order to enable different molecular modules to be connected to each other in the preset sequence, in some embodiments, when selecting a molecular module, the molecular module with a corresponding terminal portion is selected, that is, a molecular fragment serving as the terminal portion is a part of the corresponding molecular module to realize the sequential connection of different molecular modules.

In some other embodiments, combining the determined molecular modules in the preset sequence such that the composition corresponds to the information to be stored may include: forming the terminal portion corresponding to the preset sequence at a connection end of the determined molecular module; and mixing the molecular modules formed with corresponding terminal portions to generate the composition corresponding to the information to be stored. That is, the molecular fragments serving as the terminal portions are added to the corresponding molecular modules after the molecular modules are determined.

For example, during the combining process, different terminal portions may be added to the tails of the molecular modules so that they may be connected to other molecular modules respectively, so as to form the correct molecular chain.

In a specific example, the single-chain or double-chain DNA may be used as the molecular module. DNA is composed of base, deoxyribose and phosphate, wherein there are four kinds of bases in total: adenine (A), guanine (G), thymine (T) and cytosine (C). Accordingly, the terminal portion of the DNA molecule module may include the sticky end.

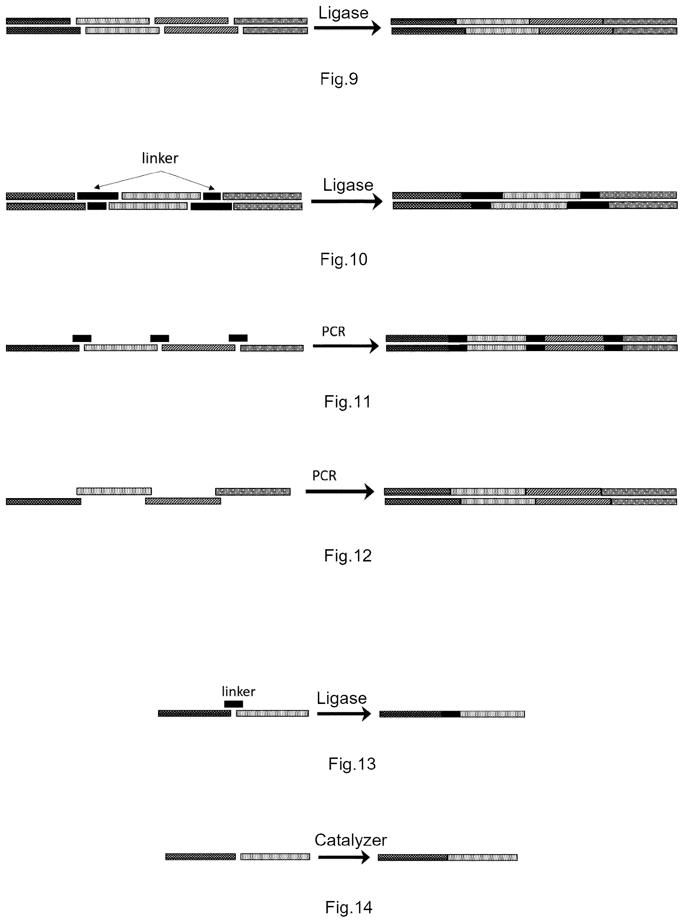

When assembling various DNAs, as shown in Fig. 9, a ligase may be used for combining multiple DNA chains in the preset sequence. Alternatively, as shown in Fig. 10, linkers disposed at ends of the DNA chains may be used for sequentially combining multiple DNA chains under the action of ligase. Alternatively, as shown in Figs. 11 and 12, the polymerase chain reaction (PCR) may be used for combining the determined DNAs in the preset sequence. In the PCR method shown in Fig. 11, the determined multiple DNA chains are served as the templates, and the DNA chains containing the corresponding sequences at the connection ends of two segments of DNA chains to be connected are amplified. In the PCR method shown in Fig. 12, the DNA chains to be connected may be directly amplified.

In another specific example, RNA may be utilized as the molecular module, wherein RNA is composed of phosphate, ribose and base, and the bases of RNA mainly include four types as follows: A (adenine), G (guanine), C (cytosine), and U (uracil). Accordingly, the terminal portion of the RNA molecule module may include a corresponding functional group.

As shown in Fig. 13, the linker sequence disposed at the end of the RNA chain and the linker sequence of DNA may be used for combining the determined multiple RNA chains in the preset sequence under the action of ligase.

In yet another specific example, peptides may be utilized as the molecular modules. The amino group of one amino acid may condense with the carboxyl group of another amino acid to form a peptide, and the resulting amide group is called the peptide bond in protein chemistry. A molecule of amino acid is the smallest, and a molecule of protein is the largest. Two or more amino acids are dehydrated and condensed to form several peptide bonds to form the peptide chain, and multiple peptide chains are folded in multi-level to form one protein molecule. Proteins are sometimes called "polypeptides". Accordingly, the terminal portion of the peptide molecule module may include a corresponding functional group. The determined peptides may be connected under the action of the catalyst to represent at least a part of the information to be stored. As shown in Fig. 14, the determined peptides may be connected under the action of the catalyst to represent at least a part of the information to be stored.

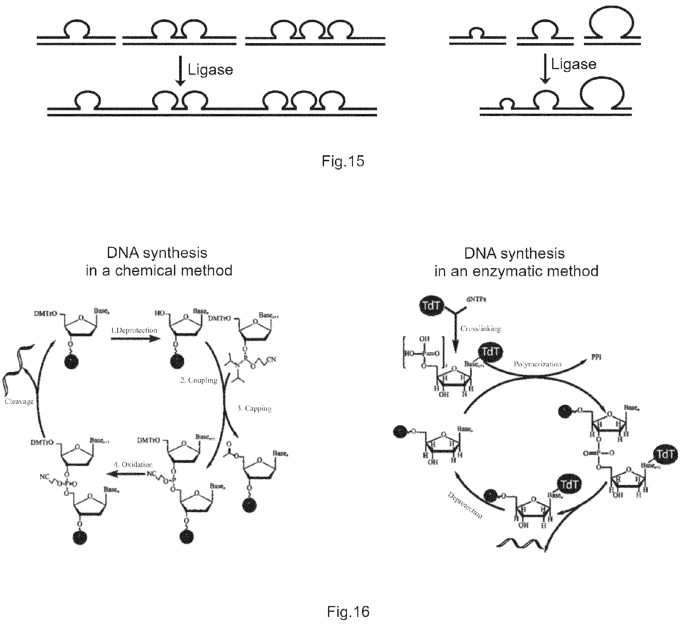

In addition, as described above, different secondary structures of the molecular modules may be used for representing different values corresponding to different target contents. In two specific examples shown in Fig. 15, the molecular modules with different secondary structures may be connected together under the action of ligases.

In some embodiments, as described above, the synthesis may be performed starting from the unit modules used for composing the molecular modules instead of the molecular modules. Taking the composition being DNA or RNA and the corresponding molecular modules being DNA fragments or RNA fragments as an example, after the molecular modules are determined according to the information to be stored, the nucleotides with corresponding bases required to combine these molecular modules may be further determined according to the determined molecular modules, and these nucleotides are obtained (for example, corresponding nucleotides are obtained by preparing, purchasing or the like); and then the composition representing the information to be stored is synthesized directly from the nucleotides by using the methods such as the chemical method, the enzymatic method or the like, as shown in Fig. 16. In this process, individual molecular modules may not actually be generated, but rather the final composition may be generated directly from the unit modules. In the chemical method, column synthesis, chip synthesis or the like based on the principle of phosphoramidite may be employed, including photochemical deprotection synthesis, electrochemical synthesis, micro-droplet method or the like. In the enzymatic method, the corresponding enzymes act as the catalysts to facilitate synthesis.

In addition, in order to facilitate reading the stored information and avoid interference in reading information caused by individual errors in the combination of molecular modules, an editing distance between different molecular modules may be greater than or equal to a preset distance threshold. The editing distance is the number of operation steps required to convert one molecular module to another molecular module. For example, when a molecular module is a DNA chain including 10 bases, the editing distance between the molecular modules "ATCGTAGCCA" and "TTCGTAGCCA" is 1, and the editing distance between the molecular modules "ATCGTAGCCA" and "TAGCATCGGT" is 10. In order to facilitate reading, when designing the molecular modules, only multiple molecular modules whose editing distance between two of them is greater than or equal to the preset distance threshold may be selected.

As such, during the process of reading information, even if there are errors in individual bases or other fragments in the molecular module, as long as it can be determined that the editing distance is less than the preset distance threshold, the read molecular module can still correspond to a specific code, thereby improving the fault tolerance of molecular storage.

Each embodiment in this specification is described in a progressive manner, and each embodiment focuses on its difference from other embodiments. The same or similar parts among the various embodiments may be the reference for each other. As for the device embodiments, since they basically correspond to the method embodiments, the description is relatively simple. For relevant details, please refer to the partial description of the method embodiments.

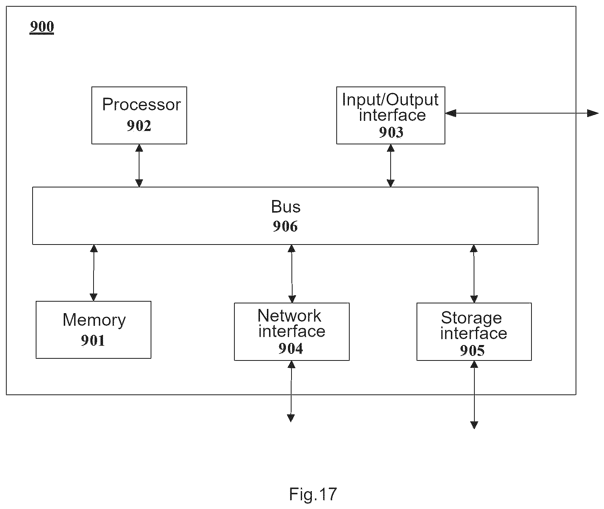

Fig. 17 is a schematic diagram of a device for storing information in a molecule according to an exemplary embodiment of the present disclosure. The device 900 may include a memory 901 and a processor 902 coupled to the memory 901. The processor 902 is configured to perform the method for storing information in the molecule as described above based on the instructions stored in the memory 901.

The memory 901 may include, for example, a system memory, a fixed non-volatile storage media, and the like. The system memory may store, for example, an operating system, an application program, a boot loader program, and other programs.

The device 900 for storing information in the molecule may further include an input and output (I/O) interface 903, a network interface 904, a storage interface 905, and the like. These interfaces 903, 904, and 905, as well as the memory 901 and the processor 902, may be connected through a bus 906, for example. The I/O interface 903 provides a connection interface for an input and output device such as a display, a mouse, a keyboard, a touch screen and the like. The network interface 904 provides a connection interface for various networked devices. The storage interface 905 provides a connection interface for an external storage device such as a SD card, a USB disk and the like.

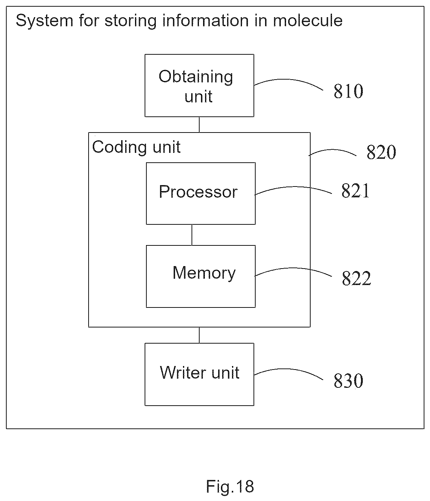

Fig. 18 is a schematic diagram of a system for storing information in a molecule according to an exemplary embodiment of the present disclosure. The system may include an obtaining unit 810, a coding unit 820, and a writer unit 830. The obtaining unit 810 may be configured to obtain the information to be stored, wherein the information to be stored has one or more bit-groups, a position of each bit-group in the information to be stored is represented by a first address, a value of each bit-group is represented by a first content, and each bit-group has one or more bits. In some embodiments, the coding unit 820 may be configured to determine a molecular module corresponding to at least one bit-group of the one or more bit-groups, wherein the molecular module includes a first molecular module, and the first molecular module is configured to represent both the first address and the first content of a corresponding bit-group. In some other embodiments, the coding unit 820 may be configured to determine one or more target bit-groups according to distribution of each kind of first content in the information to be stored; and to determine a molecular module corresponding to each target bit-group of the one or more target bit-groups, wherein the molecular module includes a third molecular module, and the third molecular module is configured to represent the first address of a corresponding target bit-group. As shown in Fig. 18, the coding unit 820 may further include a processor 821 and a memory 822 to implement corresponding coding. The writer unit 830 may be configured to generate a composition based on the determined molecular module such that the composition corresponds to the information to be stored.

The embodiments of the present disclosure further provide a computer-readable storage medium on which instructions are stored. When the instructions are executed by a processor, the method for storing information in the molecule as described above is implemented.

Similarly, the computer-readable storage media in the embodiments of the present disclosure may be volatile memory or non-volatile memory, or may include both volatile and non-volatile memories. It should be noted that computer-readable storage media described herein are intended to include, without limitation, these and any other suitable types of memory.

Embodiments of the present disclosure further provide a computer program product including instructions that, when executed by a processor, implement the method for storing information in the molecule as described above.

The instructions may be any set of instructions to be executed directly (such as machine code) or indirectly (such as a script) by one or more processors. The terms "instructions", "applications", "processes", "steps", and "procedures" are used interchangeably herein. Instructions may be stored in object code format for direct processing by one or more processors, or in any other computer language, including a script or collection of independent source code modules that are interpreted on demand or precompiled. The functionality, methods, and routines of instructions are explained in more detail elsewhere in this description.

The present disclosure is described with reference to the flowcharts and/or block diagrams of methods, devices (systems), and computer program products according to the embodiments of the present disclosure. It should be understood that the functions specified in one or more processes in the flowcharts and/or in one or more blocks in the block diagrams may be implemented by computer program instructions. These computer program instructions may be provided to a processor of a general-purpose computer, special purpose computer, embedded processor, or other programmable data processing device to produce a machine, such that the instructions executed by the processor of the computer or other programmable data processing device produce a device for realizing the functions specified in one or more processes in the flowcharts and/or one or more blocks in the block diagrams.

These computer program instructions may also be stored in a computer-readable memory that can guide a computer or other programmable data processing device to operate in a particular manner, such that the instructions stored in the computer-readable memory produce a manufacturing product including an instruction mean, and the instructions mean implements the functions specified in one or more processes in the flowcharts and/or in one or more blocks in the block diagrams.

These computer program instructions may also be loaded onto a computer or other programmable data processing device, causing a series of operating steps to be performed on the computer or other programmable device to produce computer-implemented processing, so that the instructions executed on the computer or other programmable device provide steps for implementing the functions specified in one or more processes in the flowcharts and/or in one or more blocks in the block diagrams.

Those skilled in the art will appreciate that embodiments of the present disclosure may be provided as methods, systems, or computer program products. Accordingly, the present disclosure may take the form of an entirely hardware embodiment, an entirely software embodiment, or an embodiment that combines software and hardware aspects. Furthermore, the present disclosure may take the form of a computer program product implemented on one or more computer-usable non-transitory storage media (including, but not limited to, disk memory, CD-ROM, optical storage, etc.) having computer-usable program code thereon.

Up to this point, various embodiments of the present disclosure have been described in detail. To avoid obscuring the concepts of the present disclosure, some details that are well known in the art have not been described. Based on the above description, those skilled in the art can completely understand how to implement the technical solution disclosed here.

Although some specific embodiments of the present disclosure have been described in detail with examples, those skilled in the art will understand that the above examples are for illustration only and are not intended to limit the scope of the present disclosure. Those skilled in the art should understand that the above embodiments can be modified or some technical features can be equivalently replaced without departing from the scope and spirit of the present disclosure. The scope of the present disclosure is defined by the appended claims.

**Claims**

1. A method for storing information in a molecule, comprising:

   obtaining information to be stored, wherein the information to be stored has one or more bit-groups, a position of each bit-group in the information to be stored is represented by a first address, a value of each bit-group is represented by a first content, and each bit-group has one or more bits;
   determining a molecular module corresponding to at least one bit-group of the one or more bit-groups, wherein the molecular module comprises a first molecular module, and the first molecular module is configured to represent both the first address and the first content of a corresponding bit-group; and
   generating a composition based on the determined molecular module such that the composition corresponds to the information to be stored.

2. The method according to claim 1, wherein obtaining the information to be stored comprises:

   dividing initial information to be stored to generate one or more pieces of information to be stored, wherein a number of bits of each piece of information to be stored is less than a number of bits of the initial information to be stored, and the number of bits of each piece of information to be stored is equal or not equal to each other; or
   combining a plurality of pieces of initial information to be stored to generate the information to be stored, wherein the number of bits of the information to be stored is greater than the number of bits of each piece of initial information to be stored.

3. The method according to claim 1, wherein determining the molecular module corresponding to the at least one bit-group of the one or more bit-groups comprises:
   determining the first molecular module corresponding to each bit-group of the one or more bit-groups.

4. The method according to claim 1, wherein the information to be stored has a plurality of bit-groups, and values of the plurality of bit-groups contain at least two kinds of first contents, and
   determining the molecular module corresponding to the at least one bit-group of the one or more bit-groups comprises:
   determining that a bit-group with a value of one kind of first content does not correspond to any molecule module, and determining the first molecule module corresponding to at least one bit-group of other bit-group with a value of other kind of first content.

5. The method according to claim 1, wherein determining the molecular module corresponding to the at least one bit-group of the one or more bit-groups comprises:

   determining if a second bit-group and a first bit-group of the one or more bit-groups satisfy a preset relationship, wherein the first address of the second bit-group is different from the first address of the first bit-group; and
   in a case that the second bit-group and the first bit-group satisfy the preset relationship, determining a molecular module corresponding to the second bit-group, wherein the molecular module comprises a second molecular module, and the second molecular module is configured to represent the preset relationship between the corresponding second bit-group and the first bit-group.

6. The method according to claim 5, wherein the second molecular module comprises at least one of:

   a stroke incremental module, wherein the stroke incremental module corresponding to a first preset number $a_1$ is configured to represent that the second bit-group and the first bit-group satisfy the following preset relationship: there are $a_1$ consecutive second bit-groups immediately after the first bit-group, and the first content of each second bit-group is same as the first content of the first bit-group, $a_1$ being a positive

integer;

a stroke multiple module, wherein the stroke multiple module corresponding to a second preset number $a_2$ is configured to represent that the second bit-group and the first bit-group satisfy the following preset relationship: there are $(a_2-1)$ consecutive second bit-groups immediately after the first bit-group, and the first content of each second bit-group is same as the first content of the first bit-group, $a_2$ being a positive integer greater than 1;

a stroke flip module, wherein the stroke flip module corresponding to a third preset number $a_3$ is configured to represent that the second bit-group and the first bit-group satisfy the following preset relationship: each bit in the first bit-group and the second bit-group has a value of 0 or 1 only, the second bit-group has two bits with different values only, there are $a_3$ consecutive second bit-groups immediately after the first bit-group, and a value of a first bit of the second bit-group is different from a value of a last bit of the first bit-group, $a_3$ being a positive integer;

a stroke repeat module, wherein the stroke repeat module is configured to represent that the second bit-group and the first bit-group satisfy the following preset relationship: the first content of the second bit-group is same as the first content of the first bit-group;

a bit increase module, wherein the bit increase module corresponding to a fourth preset number $a_4$ is configured to represent that the second bit-group and the first bit-group satisfy the following preset relationship: the second bit-group has one bit only, there are $a_4$ consecutive second bit-groups immediately after the first bit-group, and a value of the second bit-group is same as the value of the last bit of the first bit-group, $a_4$ being a positive integer; or

a bit decrease module, wherein the bit decrease module corresponding to a fifth preset number as is configured to represent that the second bit-group and the first bit-group satisfy the following preset relationship: as consecutive bits from back to front in the first bit-group are determined as the second bit-group, and the second bit-group in the first bit-group is removed, $a_5$ being a positive integer and $a_5$ being less than or equal to a total number of bits of the first bit-group.

7. The method according to claim 1, wherein determining the molecular module corresponding to the at least one bit-group of the one or more bit-groups comprises:

converting at least part of the information to be stored from a first system of numeration to a second system of numeration;

dividing the at least part of the information to be stored after conversion into one or more bit-groups; and

determining a molecular module corresponding to at least one bit-group of the one or more bit-groups obtained after conversion.

8. The method according to claim 7, wherein the first system of numeration is binary, the second system of numeration is decimal, and converting the at least part of the information to be stored from the first system of numeration to the second system of numeration comprises:

performing an iterative calculation according to $x_{i+1} = 2x_i + b_{i+1}$ to determine the at least part of the information to be stored in decimal, wherein $x_0$ is a preset initial value, $b_{i+1}$ is a value of $(i+1)$th bit from left to right in the at least part of the information to be stored in binary, $i$ being an integer greater than or equal to 0, and in a case that $(i+1)$ is equal to a total number of bits of the at least part of the information to be stored in binary, a corresponding $x_{i+1}$ is the at least part of the information to be stored in decimal.

9. The method according to claim 7, wherein the first system of numeration is binary, the second system of numeration is decimal, and converting the at least part of the information to be stored from the first system of numeration to the second system of numeration comprises:

calculating the at least part of the information to be stored in decimal according to

$$x = \sum_{n=0}^{i} 2^n * a_n$$

, wherein $a_n$ is a value of $(n+1)$th bit in the at least part of the information to be stored in binary, and $(i+1)$ is a total number of bits of the at least part of the information to be stored in binary.

10. The method according to claim 7, wherein the first system of numeration is binary, the second system of numeration is an integer multiple of two base, and converting the at least part of the information to be stored from the first system of numeration to the second system of numeration comprises:

determining whether the at least part of the information to be stored needs to be padded at a preset position according to the second system of numeration and a total number of bits of the at least part of the information to be stored,

if so, padding the at least part of the information to be stored according to the second system of numeration to obtain target information to be stored,

if not, directly taking the at least part of the information to be stored as the target information to be stored;

dividing the at least part of the information to be stored in binary into one or more pieces of sub-information according to the second system of numeration, wherein a number of bits of each piece of sub-information is an integer multiple of the second system of numeration with respect to the binary; and

converting each piece of sub-information in binary into a corresponding value in the second system of numeration to generate the at least part of the information to be stored in the second system of numeration.

11. The method according to claim 1, wherein a relative position of the determined molecular module in the composition is consistent with a relative position of the corresponding bit-group in the information to be stored.

12. The method according to claim 1, wherein generating the composition based on the determined molecular module such that the composition corresponds to the information to be stored comprises:

obtaining a unit module corresponding to the determined molecular module, wherein each molecular module is synthesized from one or more unit modules; and

synthesizing the obtained unit module to generate the composition corresponding to the information to be stored.

13. The method according to claim 12, wherein the determined molecular module comprises at least one of a DNA fragment or an RNA fragment, and the unit module comprises a nucleotide,

synthesizing the obtained unit module to generate the composition corresponding to the information to be stored comprises:

synthesizing the composition directly starting from the obtained nucleotide, wherein at least a fragment of the composition is consistent with the determined molecular module.

14. The method according to claim 1, wherein generating the composition based on the determined molecular module such that the composition corresponds to the information to be stored comprises:

forming a terminal portion corresponding to a preset sequence at a connection end of the determined molecular module; and

mixing the molecular modules formed with the corresponding terminal portions to generate the composition corresponding to the information to be stored.

15. The method according to claim 14, wherein a molecular fragment as the terminal portion is part of the corresponding molecular module; or

the molecular fragment as the terminal portion is added to the corresponding molecular module after the molecular module is determined.

16. The method according to claim 14, wherein mixing the molecular modules formed with the corresponding terminal portions to generate the composition corresponding to the information to be stored comprises at least one of:

combining the determined molecular modules in the preset sequence by using a ligase;

combining the determined molecular modules in the preset sequence by using linkers disposed at ends of the molecular modules; or

combining the determined molecular modules in the preset sequence by using a polymerase chain reaction.

17. The method according to claim 1, wherein the molecular module comprises at least one of: a deoxyribonucleic acid, a ribonucleic acid, a non-natural nucleotides, a modified nucleotides, an artificially synthetic nucleotide, a peptide, an organic polymer, an organic small molecule, a carbon nanomaterial, an inorganic substance, or spaced molecular fragments.

18. The method according to claim 1, wherein various molecular modules are distinguished by at least one of: a sequence distribution, a sequence length, a secondary structure, a crystalline or amorphous nature or a morphology of the molecular modules.

19. The method according to claim 1, wherein an edit distance between different molecular modules is greater than or equal to a preset distance threshold.

20. A method for storing information in a molecule, comprising:

obtaining information to be stored, wherein the information to be stored has one or more bit-groups, a position of each bit-group in the information to be stored is represented by a first address, a value of each bit-group is represented by a first content, and each bit-group has one or more bits;

determining one or more target bit-groups according to distribution of each kind of first content in the information to be stored;

determining a molecular module corresponding to each target bit-group of the one or more target bit-groups, wherein the molecular module comprises a third molecular module, and the third molecular module is configured to represent the first address of a corresponding target bit-group; and

generating a composition based on the determined molecular module such that the composition corresponds to the information to be stored.

21. The method according to claim 20, wherein obtaining the information to be stored comprises:

dividing initial information to be stored to generate one or more pieces of information to be stored, wherein a number of bits of each piece of information to be stored is less than a number of bits of the initial information to be stored, and the number of bits of each piece of information to be stored is equal or not equal to each other; or combining a plurality of pieces of initial information to be stored to generate the information to be stored, wherein the number of bits of the information to be stored is greater than the number of bits of each piece of initial information to be stored.

22. The method according to claim 20, wherein determining the one or more target bit-groups according to the distribution of each kind of first content in the information to be stored comprises:

counting an occurrence frequency of each kind of first content in the information to be stored, and determining a most frequent content with a highest occurrence frequency; and determining a bit-group with a value other than the most frequent content as the target bit-group.

23. The method according to claim 20, wherein the third molecular module comprises one or more sub-molecule modules, and determining the molecular module corresponding to each target bit-group of the one or more target bit-groups comprises:

dividing the first address of the target bit-group into one or more address bit-groups, wherein a position of each address bit-group in the first address of the target bit-group is represented by a second address, a value of each address bit-group is represented by a second content, and each address bit-group has one or more bits; determining a sub-molecule module corresponding to at least one address bit-group of the one or more address bit-groups, wherein the sub-molecule module comprises a first sub-molecule module, and the first sub-molecule module is configured to represent both the second address and the second content of a corresponding address bit-group; and determining the third molecular module based on a combination of the determined sub-molecule module.

24. The method according to claim 23, wherein determining the sub-molecule module corresponding to the at least one address bit-group of the one or more address bit-groups comprises:

determining if a second address bit-group and a first address bit-group of the one or more address bit-groups satisfy a preset relationship, wherein the second address of the second address bit-group is different from the second address of the first address bit-group; and in a case that the second address bit-group and the first address bit-group satisfy the preset relationship, determining the sub-molecule module corresponding to the second address bit-group, wherein the sub-molecule module comprises a second sub-molecule module, and the second sub-molecule module is configured to represent the preset relationship between the corresponding second address bit-group and the first address bit-group.

25. The method according to claim 24, wherein the second sub-molecule module comprises:
a stroke increment module, wherein the stroke increment module corresponding to a sixth preset number $a_6$ is configured to represent that the second address bit-group of the second bit-group and the first address bit-group of the first bit-group satisfy the following preset relationship: a sum of the first address bit-group and $a_6$ is the second address bit-group, and the first content of the second bit-group is same as the first content of the first bit-group.

26. The method according to claim 20, wherein the molecular module further comprises a fourth molecular module, and the fourth molecular module is configured to represent the first content of the corresponding target bit-group.

27. The method according to claim 20, wherein determining the molecular module corresponding to each target bit-group of the one or more target bit-groups comprises:

converting at least part of the information to be stored from a first system of numeration to a second system of numeration; dividing the at least part of the information to be stored after conversion into one or more bit-groups; and determining a molecular module corresponding to at least one bit-group of the one or more bit-groups obtained after conversion.

28. The method according to claim 27, wherein the first system of numeration is binary, the second system of numeration is decimal, and converting the at least part of the information to be stored from the first system of numeration to the second system of numeration comprises:
performing an iterative calculation according to $x_{i+1} = 2x_i + b_{i+1}$ to determine the at least part of the information to be stored in decimal, wherein $x_0$ is a preset initial value, $b_{i+1}$ is a value of $(i+1)$th bit from left to right in the at least part of the information to be stored in binary, i being an integer greater than or equal to 0, and in a case that $(i+1)$ is equal to a total number of bits of the at least part of the information to be stored in binary, a corresponding $x_{i+1}$ is the at least part of the information to be stored in decimal.

29. The method according to claim 27, wherein the first system of numeration is binary, the second system of numeration is decimal, and converting the at least part of the information to be stored from the first system of numeration to the second system of numeration comprises:
calculating the at least part of the information to be stored in decimal according to $$x = \sum_{n=0}^{i} 2^n * a_n$$, wherein $a_n$ is a value of $(n+1)$th bit in the at least part of the information to be stored in binary, and $(i+1)$ is a total number of bits of the at least part of the information to be stored in binary.

30. The method according to claim 27, wherein the first system of numeration is binary, the second system of numeration is an integer multiple of two base, and converting the at least part of the information to be stored from the first system of numeration to the second system of numeration comprises:
determining whether the at least part of the information to be stored needs to be padded at a preset position according to the second system of numeration and a total number of bits of the at least part of the information to be stored,

if so, padding the at least part of the information to be stored according to the second system of numeration to obtain target information to be stored,
if not, directly taking the at least part of the information to be stored as the target information to be stored;
dividing the at least part of the information to be stored in binary into one or more pieces of sub-information according to the second system of numeration, wherein a number of bits of each piece of sub-information is an integer multiple of the second system of numeration with respect to the binary; and
converting each piece of sub-information in binary into a corresponding value in the second system of numeration to generate the at least part of the information to be stored in the second system of numeration.

31. The method according to claim 20, wherein a relative position of the determined molecular module in the composition is consistent with a relative position of the corresponding bit-group in the information to be stored.

32. The method according to claim 20, wherein generating the composition based on the determined molecular module such that the composition corresponds to the information to be stored comprises:

obtaining a unit module corresponding to the determined molecular module, wherein each molecular module is synthesized from one or more unit modules; and
synthesizing the obtained unit module to generate the composition corresponding to the information to be stored.

33. The method according to claim 32, wherein the determined molecular module comprises at least one of a DNA fragment or an RNA fragment, and the unit module comprises a nucleotide,
synthesizing the obtained unit module to generate the composition corresponding to the information to be stored comprises:
synthesizing the composition directly starting from the obtained nucleotide, wherein at least a fragment of the composition is consistent with the determined molecular module.

34. The method according to claim 20, wherein generating the composition based on the determined molecular module such that the composition corresponds to the information to be stored comprises:

forming a terminal portion corresponding to a preset sequence at a connection end of the determined molecular module; and
mixing the molecular modules formed with the corresponding terminal portions to generate the composition corresponding to the information to be stored.

35. The method according to claim 34, wherein a molecular fragment as the terminal portion is part of the corresponding molecular module; or
the molecular fragment as the terminal portion is added to the corresponding molecular module after the molecular module is determined.

36. The method according to claim 34, wherein mixing the molecular modules formed with the corresponding terminal portions to generate the composition corresponding to the information to be stored comprises at least one of:

combining the determined molecular modules in the preset sequence by using a ligase;
combining the determined molecular modules in the preset sequence by using linkers disposed at ends of the molecular modules; or
combining the determined molecular modules in the preset sequence by using a polymerase chain reaction.

37. The method according to claim 20, wherein the molecular module comprises at least one of: a deoxyribonucleic acid, a ribonucleic acid, a non-natural nucleotides, a modified nucleotides, an artificially synthetic nucleotide, a peptide, an organic polymer, an organic small molecule, a carbon nanomaterial, an inorganic substance, or spaced molecular fragments.

38. The method according to claim 20, wherein various molecular modules are distinguished by at least one of: a sequence distribution, a sequence length, a secondary structure, a crystalline or amorphous nature or a morphology of the molecular modules.

39. The method according to claim 20, wherein an edit distance between different molecular modules is greater than or equal to a preset distance threshold.

40. A device for storing information in a molecule, comprising:

a memory having instructions stored thereon; and
a processor coupled to the memory, wherein the instructions, when executed by the processor, implement the method according to any one of claims 1-39.

41. A system for storing information in a molecule, comprising:

an obtaining unit configured to obtain information to be stored, wherein the information to be stored has one or more bit-groups, a position of each bit-group in the information to be stored is represented by a first address, a value of each bit-group is represented by a first content, and each bit-group has one or more bits;
a coding unit configured to determine a molecular module corresponding to at least one bit-group of the one or more bit-groups, wherein the molecular module comprises a first molecular module, and the first molecular module is configured to represent both the first address and the first content of a corresponding bit-group; and
a writer unit configured to generate a composition based on the determined molecular module such that the composition corresponds to the information to be stored.

42. A system for storing information in a molecule, comprising:

an obtaining unit configured to obtain information to be stored, wherein the information to be stored has one or more bit-groups, a position of each bit-group in the information to be stored is represented by a first address, a value of each bit-group is represented by a first content, and each bit-group has one or more bits;
a coding unit configured to determine one or more target bit-groups according to distribution of each kind of first content in the information to be stored; and to determine a molecular module corresponding to each target bit-group of the one or more target bit-groups, wherein the molecular module comprises a third molecular module, and the third molecular module is configured to represent the first address of a corresponding target bit-group; and
a writer unit configured to generate a composition based on the determined molecular module such that the composition corresponds to the information to be stored.

43. A computer-readable storage medium having instructions stored thereon, wherein the instructions, when executed by a processor, implement the method according to any one of claims 1-39.

44. A computer program product comprising instructions that, when executed by a processor, implement the method according to any one of claims 1-39.

Obtaining information to be stored

S100

Determining a molecular module corresponding to
at least one bit-group of the one or more bit-groups

S210

Generating a composition based on the determined
molecular module such that the composition
corresponds to the information to be stored

S300

Fig.1

| m+2ⁿ \ 2ⁿ / m | ... | $2^9$-bit | ... | $2^3$-bit | $2^2$-bit | $2^1$-bit | $2^0$-bit |
|---|---|---|---|---|---|---|---|
| 0 | ... | ◈ | ... | ▨ | ⬡ | ⬤ | ⬡ |
| 1 | ... | ◇ | ... | ▨ | ⬡ | ◯ | ⬡ |

1010

Fig.2(a)

Fig.2(b)

Fig.3(a)

1 0 1 1 1 0

2 3 4

2|+1 +1

2| ×3

Fig.3(b)

1 0 1 0 1

0| +01 +01

Fig.3(c)

0 1 1 1 0 0 1 1 1 0

1 2 3    6 7 8

the first ×3    the sixth    repeating
bit              bit         the first bit
                             to the third bit

Fig.4(a)

0 1 1 1 0 0 1 1 1 1 0

1 2 3    6 7 8 9

the sixth    repeating    adding
bit          the first bit  one bit
             to the third bit

Fig.4(b)

0 1 1 1 0 0 1 1 0

1 2 3    6 7

the sixth   repeating   subtracting
bit    the first bit    one bit
to the third bit

Fig.4(c)

| | |
|---|---|
| Obtaining information to be stored | S100 |
| Determining one or more target bit-groups according to distribution of each kind of first content in the information to be stored | S221 |
| Determining a molecular module corresponding to each target bit-group of the one or more target bit-groups | S222 |
| Generating a composition based on the determined molecular module such that the composition corresponds to the information to be stored | S300 |

Fig.5

$$0\ 0\ 0\ 0\ 1\ 0\ 0\ 0\ \ldots\ldots\ 1\ \ldots\ldots\ 1\ \ldots\ldots$$

$$\uparrow \qquad\qquad\quad \uparrow \qquad\quad \uparrow$$

$$4 \qquad\qquad\quad 5371 \qquad 10^9$$

Fig.6(a)

$4 =$

$4 \times 10^0$

$10^0$-bit
(4)

$5371 =$

$5 \times 10^3 + 3 \times 10^2 + 7 \times 10^1 + 1 \times 10^0$

$10^3$-bit $\quad$ $10^2$-bit $\quad$ $10^1$-bit $\quad$ $10^0$-bit
(5) $\qquad$ (3) $\qquad$ (7) $\qquad$ (1)

$10^9 =$

$1 \times 10^9 + 0 \times 10^8 + 0 \times 10^7 + 0 \times 10^6 + 0 \times 10^5$
$+ 0 \times 10^4 + 0 \times 10^3 + 0 \times 10^2 + 0 \times 10^1 + 0 \times 10^0$

$10^9$-bit $\qquad\qquad$ $10^3$-bit $\quad$ $10^2$-bit $\quad$ $10^1$-bit $\quad$ $10^0$-bit
(1) $\qquad\qquad\qquad$ (0) $\qquad$ (0) $\qquad$ (0) $\qquad$ (0)

Fig.6(b)

|   | Increment the first bit | Increment the second bit | Increment the third bit |
|---|---|---|---|
| 0 | | | |
| 1 | | | |
| 2 | | | |
| 3 | | | |
| 4 | | | |
| 5 | | | |
| 6 | | | |
| 7 | | | |
| 8 | | | |
| 9 | | | |

Fig.7(a)

0 1 1 0 0 1 0 0 0 1

1 2 5 9

1|+1 + 3 + 4

Fig.7(b)

0 1 1 1 0 1 0 0 0 1

↑ ↑ ↑   ↑       ↑

1 2 3   5       9

$$1|×3 + 2 + 4$$

Fig.7(c)

1 0 0 1 1 0 1 0 1 0
100    103 104 106 108

100

103

104

106

108

Fig.8(a)

1001101010 ⟶ 1642

Binary       Natural number

Starting position100    Converted natural number1642

Fig.8(b)

Fig.9

linker

Ligase

Fig.10

PCR

Fig.11

PCR

Fig.12

linker

Ligase

Fig.13

Catalyzer

Fig.14

Fig.15

DNA synthesis
in a chemical method

DNA synthesis
in an enzymatic method

Fig.16

**900**

```
┌─────────────────────────────────────────────────────────────────┐
│ 900                                                               │
│                                                                   │
│    ┌──────────────┐              ┌──────────────┐                 │
│    │  Processor   │              │ Input/Output │                 │
│    │     902      │              │  interface   │◄──────────────► │
│    │              │              │     903      │                 │
│    └──────┬───────┘              └──────┬───────┘                 │
│           ▲                             ▲                         │
│           ▼                             ▼                         │
│    ┌──────────────────────────────────────────────────────┐      │
│    │                      Bus                              │      │
│    │                      906                              │      │
│    └──┬──────────────────────┬────────────────────┬───────┘      │
│       ▲                      ▲                     ▲              │
│       ▼                      ▼                     ▼              │
│  ┌─────────┐         ┌──────────────┐      ┌──────────────┐       │
│  │ Memory  │         │   Network    │      │   Storage    │       │
│  │   901   │         │  interface   │      │  interface   │       │
│  │         │         │     904      │      │     905      │       │
│  └─────────┘         └──────┬───────┘      └──────┬───────┘       │
│                             ▲                     ▲               │
└─────────────────────────────┼─────────────────────┼──────────────┘
                              ▼                     ▼
```

Fig.17

System for storing information in molecule

Obtaining unit — 810

Coding unit — 820

Processor — 821

Memory — 822

Writer unit — 830

Fig.18

## INTERNATIONAL SEARCH REPORT

| International application No. |
| --- |
| **PCT/CN2022/119796** |

| A. | CLASSIFICATION OF SUBJECT MATTER |
| --- | --- |

G06N 3/12(2023.01)i

According to International Patent Classification (IPC) or to both national classification and IPC

| B. | FIELDS SEARCHED |
| --- | --- |

Minimum documentation searched (classification system followed by classification symbols)

IPC: G06N

Documentation searched other than minimum documentation to the extent that such documents are included in the fields searched

Electronic data base consulted during the international search (name of data base and, where practicable, search terms used)

DWPI, CNTXT, USTXT, ENTXTC, CNKI, IEEE, WOTXT: 分子, 存储, 信息, 数据, 位组, 位, 地址, 位置, 编码, 二进制, 组合物, 内容, 合并, data, address, locat+, stor+, position, information, molecules, encod+, binary, digit, dna

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 112771215 A (PRESIDENT AND FELLOWS OF HARVARD COLLEGE et al.) 07 May 2021 (2021-05-07) <br> description, paragraphs [0032]-[0078] | 1-44 |
| A | CN 111489791 A (CHONGQING INSTITUTE OF GREEN AND INTELLIGENT TECHNOLOGY, CHINESE ACADEMY OF SCIENCES et al.) 04 August 2020 (2020-08-04) <br> entire document | 1-44 |
| A | CN 110569974 A (TIANJIN UNIVERSITY) 13 December 2019 (2019-12-13) <br> entire document | 1-44 |
| A | EP 2947779 A1 (THOMSON LICENSING) 25 November 2015 (2015-11-25) <br> entire document | 1-44 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 April 2023** | **20 April 2023** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
Information on patent family members

International application No.

**PCT/CN2022/119796**

| Patent document cited in search report | | | Publication date (day/month/year) | Patent family member(s) | | | Publication date (day/month/year) |
|---|---|---|---|---|---|---|---|
| CN | 112771215 | A | 07 May 2021 | US | 2021217474 | A1 | 15 July 2021 |
| | | | | EP | 3856958 | A2 | 04 August 2021 |
| | | | | WO | 2020112233 | A2 | 04 June 2020 |
| | | | | JP | 2022508024 | A | 19 January 2022 |
| CN | 111489791 | A | 04 August 2020 | None | | | |
| CN | 110569974 | A | 13 December 2019 | None | | | |
| EP | 2947779 | A1 | 25 November 2015 | WO | 2015176990 | A1 | 26 November 2015 |

Form PCT/ISA/210 (patent family annex) (July 2022)

**EP 4 575 914 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210988065